(19) Europäisches Patentamt
European Patent Office
Office européen des brevets

(11) EP 1 754 479 A1

(12) EUROPEAN PATENT APPLICATION
published in accordance with Art. 158(3) EPC

(43) Date of publication:
**21.02.2007 Bulletin 2007/08**

(21) Application number: **05751213.9**

(22) Date of filing: **10.06.2005**

(51) Int Cl.:
***A61K 31/20*** *(2006.01)* ***A61K 9/48*** *(2006.01)*
***A61K 47/10*** *(2006.01)* ***A61K 47/42*** *(2006.01)*
***A61P 25/00*** *(2006.01)*

(86) International application number:
**PCT/JP2005/011092**

(87) International publication number:
**WO 2005/120490 (22.12.2005 Gazette 2005/51)**

(84) Designated Contracting States:
**AT BE BG CH CY CZ DE DK EE ES FI FR GB GR HU IE IS IT LI LT LU MC NL PL PT RO SE SI SK TR**

(30) Priority: **11.06.2004 JP 2004174576**
**20.04.2005 JP 2005122821**

(71) Applicant: **ONO PHARMACEUTICAL CO., LTD.**
**Osaka-shi, Osaka 541-8526 (JP)**

(72) Inventors:
- **OKAMOTO, Ichiro**
  **c/o Ono Pharmaceutical Co., Ltd.**
  **Mishima-gun, Osaka 618-8585 (JP)**
- **MIYAMOTO, Yuji**
  **c/o Ono Pharmaceutical Co., Ltd.**
  **Mishima-gun, Osaka 618-8585 (JP)**
- **NISHIMURA, Hidekatsu**
  **Ono Pharmaceutical Co., Ltd.**
  **Mishima-gun, Osaka 618-8585 (JP)**

(74) Representative: **Henkel, Feiler & Hänzel**
**Patentanwälte**
**Maximiliansplatz 21**
**80333 München (DE)**

# (54) CAPSULE STABLE AGAINST MASTICATION

(57) The present invention relates to a soft capsule which is easily disintegrated in the stomach, wherein the contents thereof are not easily leaked at the time of mastication, which is obtained by providing a soft capsule comprising (2R)-2-propyloctanoic acid or a salt thereof with at least one property, preferably all properties, selected from (A) wherein it has a strength of 150 to 400 N by a cracking test; (B) wherein it has a disintegration time of 3 to 10 minutes by the disintegration test stipulated in Japanese Pharmacopoeia; (C) wherein the capsule shell has a shell thickness of 0.05 to 0.50 mm; (D) wherein the capsule shell has a first seam thickness of 0.10 to 0.55 mm; (E) wherein the capsule shell has a second seam thickness of 0.05 to 0.50 mm; (F) wherein the capsule shell has a water content of 5.0 to 9.0%.



Printed by Jouve, 75001 PARIS (FR)

## Description

TECHNICAL FIELD

The present invention relates to a capsule which comprises an oral irritative compound, wherein the contents thereof are not easily leaked at the time of mastication.

BACKGROUND ART

Expect for particular preparations such as chewable tablets and sublingual tablets, pharmaceutical preparations which are orally administered such as tablets and capsules are generally taken using water or the like without mastication. In the case of the aged and the like, however, many cases have been reported in which in taking these pharmaceutical preparations, these are masticated and crushed with the teeth intentionally or not intentionally. Since the method for internally taking the pharmaceutical preparations is peculiar to respective preparations which have been designed based on the absorbing region of the active ingredient, its blood duration and the like, when its taking method is mistaken, the desired effect cannot be obtained but it results in a serious side effect in some cases. Accordingly, although proper guidance on the taking method is carried out for patients in medical institutions such as hospitals and pharmacies, it cannot be said that its results are favorable so that there still are many patients who mistake the taking method.

A soft capsule is generally prepared by encapsulating an inclusion liquid in the state of liquid or suspension in a capsule shell made of gelatin as the base. The soft capsule is regarded as a dosage form from which proper absorption of its drug can be expected, because after its taking, the capsule shell is quickly disintegrated in the digestive tracts so that dispersion and dissolution of the inclusion liquid is carried out quicker than the case of a tablet. However, since the main purpose of the pharmaceutical preparations produced for utilizing this property of soft capsule is the easy disintegration in the digestive tracts, the capsule shell thickness is generally thin so that there is a problem that the inclusion liquid is easily leaked in the mouth when masticated by mistake at the time of taking as described in the above. Particularly, when the inclusion liquid is an oral irritative substance, it cannot be said that such a soft capsule from which the inclusion liquid is easily leaked by mastication is desirable, because it causes unpleasant senses such as acridness, pain, burning sensation and the like.

On the other hand, for example, there are the following reports regarding a capsule comprising a pentanoic acid derivative including (2R)-2-propyloctanoic acid, which is effective as a cerebral function improving agent for various diseases because of the action to improve function of astrocyte.

For example, EP0632008 describes that such a pentanoic acid derivative is administered as a solid composition for oral administration of tablets, pills, capsules, powders, granules and the like, and that hard capsules and soft capsules are included in said capsules.

In addition, for example in EP1174131, there is described a preparation example stating that 10 capsules each containing 100 mg of the active ingredient were obtained by encapsulating (2R)-2-propyloctanoic acid (1 g) in a gelatin capsule.

To date, however, there is no specific disclosure about a soft capsule, particularly a soft capsule stable to mastication, which comprises (2R)-2-propyloctanoic acid or a salt thereof

DISCLOSURE OF THE INVENTION

In order to produce a soft capsule which comprises (2R)-2-propyloctanoic acid or a salt thereof, the present inventors have carried out examinations using (2R)-2-propyloctanoic acid which is a liquid and found as a result that, being an extremely irritative substance, when (2R)-2-propyloctanoic acid is encapsulated in a soft capsule of general formulation and masticated, it is easily leaked into the oral cavity to cause considerably strong acridness, burning sensation and the like. Although (2R)-2-propyloctanoic acid is a substance expected to be administered to aged patients because of the indication, such preparations from which the contents are easily leaked when masticated are not desirable for their use in aged patients who frequently mistake the taking method of pharmaceutical preparations.

Accordingly, an object of the present invention is to provide a soft capsule which comprises (2R)-2-propyloctanoic acid or a salt thereof, wherein the contents thereof are not easily leaked even at the time of mastication.

The inventors have conducted intensive studies with the aim of solving the above-described problems and succeeded as a result in providing a property of easy disintegration in the stomach and hard leakage of the contents at the time of mastication, by producing a soft capsule which comprises (2R)-2-propyloctanoic acid or a salt thereof using a capsule shell containing gelatin and glycerine, and providing such a capsule with one or more of the characteristics selected from the (A) to (F) which are described later, preferably all of these characteristics. The inventors have further carried out examinations based on this knowledge and found that such a means can be applied not only to (2R)-2-propyloctanoic acid or a salt thereof but also to the whole compounds which have oral irritative properties, thereby

accomplishing the present invention.

That is, the present invention relates to the followings:

[1] A capsule stable against mastication comprising (2R)-2-propyloctanoic acid or a salt thereof and having a property of following (1) and/or (2):

(1) having a capsule shell comprising (a) at least one capsule base selected from a protein, a saccharide, a biodegradable plastic and a hydrogenated fat and (b) a plasticizer, and having a water content of 4.0 to 10.0%;
(2) being easily disintegrated in the stomach.

[2] The capsule stable against mastication according to [1], which is a soft capsule.
[3] The soft capsule stable against mastication according to [2], which has at least one property selected from the following (A) to (F):

(A) wherein it has a strength of 150 to 400 N by a cracking test;
(B) wherein it has a disintegration time of 3 to 10 minutes by the disintegration test stipulated in Japanese Pharmacopoeia;
(C) wherein the capsule shell has a shell thickness of 0.05 to 0.50 mm;
(D) wherein the capsule shell has a first seam thickness af 0.10 to 0.55 mm;
(E) wherein the capsule shell has a second seam thickness of 0.05 to 0.50 mm;
(F) wherein the capsule shell has a water content of 5.0 to 9.0%.

[4] The soft capsule stable against mastication according to [3], wherein the property is at least one selected from the following (A) to (F):

(A) wherein it has a strength of 180 to 350 N by a cracking test;
(B) wherein it has a disintegration time of 5 to 8 minutes by the disintegration test stipulated in Japanese Pharmacopoeia;
(C) wherein the capsule shell has a shell thickness of 0.10 to 0.45 mm;
(D) wherein the capsule shell has a first seam thickness of 0.15 to 4.50 mm;
(E) wherein the capsule shell has a second seam thickness of 0.10 to 0.40 mm;
(F) wherein the capsule shell has a water content of 5.0 to 8.0%.

[5] The soft capsule stable against mastication according to [2], wherein the capsule base is gelatin.
[6] The soft capsule stable against mastication according to [2], wherein the plasticizer is glycerine which contains substantially no sorbitol or contains sorbitol in an amount of 20 parts by mass or less relative to 100 parts by mass of the capsule base.
[7] The soft capsule stable against mastication according to [6], wherein the plasticizer is glycerine which contains substantially no sorbitol.
[8] The soft capsule stable against mastication according to [2], wherein the capsule shell comprises gelatin as the capsule base and glycerine as the plasticizer, which is contained in an amount of 25 to 40 parts by mass relative to 100 parts by mass of the gelatin, and has a water content of 5.0 to 9.0%.
[9] The soft capsule stable against mastication according to [8], wherein the capsule shell comprises gelatin and glycerine which is contained in an amount of 30 parts by mass relative to 100 parts by mass of the gelatin, and has a water content of 5.0 to 8.0%.
[10] A soft capsule stable against mastication which is easily disintegrated in the stomach, comprises (2R)-2-propyloctanoic acid, and is characterized by:

having a capsule shell comprising gelatin and glycerin which is contained in an amount of 30 parts by mass relative to 100 parts by mass of the gelatin, and having a water content of 5.0 to 8.0%, a shell thickness of 0.10 to 0.45 mm, a first seam thickness of 0.15 to 0.50 mm and a second seam thickness of 0.10 to 0.40 mm;
having a strength of 180 to 350 N by a cracking test; and
having a disintegration time of 5 to 8 minutes by the disintegration test stipulated in Japanese Pharmacopoeia.

[11] The soft capsule stable against mastication according to [2] or [10], which comprises 50 to 400 mg of (2R)-2-propyloctanoic acid.
[12] The soft capsule stable against mastication according to [11], which comprises 100 mg or 300 mg of (2R)-2-propyloctanoic acid.

[13] The soft capsule stable against mastication according to [2] or [10], wherein dissolution delay accompanied by preservation is reduced.
[14] The soft capsule stable against mastication according to [13], which has a dissolution rate of 90% or more 30 minutes after commencement of the dissolution test according to the second dissolution test stipulated in Japanese Pharmacopoeia, after preservation at room temperature for about one and half years.
[15] The soft capsule stable against mastication according to [2] or [10], which is subj ected to hermetically sealed packaging.
[16] The soft capsule stable against mastication according to [15], wherein the hermetically sealed packaging is PTP packaging.
[17] The soft capsule stable against mastication according to [2] or [10], which is an agent for prevention, treatment and/or suppression of symptom progression for neurodegenerative diseases, neuropathies or diseases in need of nerve regeneration.
[18] A method for prevention, treatment and/or suppression of symptom progression for neurodegenerative diseases, neuropathies or diseases in need of nerve regeneration, which comprises administering to a mammal an effective amount of the soft capsule stable against mastication according to [2] or [10].
[19] Use of the soft capsule stable against mastication according to [2] or [10] for the manufacture of an agent for prevention, treatment and/or suppression of symptom progression for neurodegenerative diseases, neuropathies or diseases in need of nerve regeneration.
[20] An agent for prevention, treatment and/or suppression of symptom progression for neurodegenerative diseases, neuropathies or diseases in need of nerve regeneration, which comprises the soft capsule stable against mastication according to [2] or [10].
[21] A method for stabilizing a soft capsule against mastication, which comprises providing a soft capsule comprising (2R)-2-propyloctanoic acid or a salt thereof and having a capsule shell comprising (a) at least one capsule base selected from a protein, a saccharide, a biodegradable plastic and a hydrogenated fat and (b) a plasticizer, with at least one property selected from the following (A) to (E):

(A) wherein it has a strength of 150 to 400 N by a cracking test;
(B) wherein the capsule shell has a shell thickness of 0.05 to 0.50 mm;
(C) wherein the capsule shell has a first seam thickness of 0.10 to 0.55 mm;
(D) wherein the capsule shell has a second seam thickness of 0.05 to 0.50 mm;
(E) wherein the capsule shell has a water content of 5.0 to 9.0%.

[22] The method according to [21], wherein the property is at least one selected from the following (A) to (E):

(A) wherein it has a strength of 180 to 350 N by a cracking test;
(B) wherein the capsule shell has a shell thickness of 0.10 to 0.45 mm;
(C) wherein the capsule shell has a first seam thickness of 0.15 to 0.50 mm;
(D) wherein the capsule shell has a second seam thickness of 0.10 to 0.40 mm;
(E) wherein the capsule shell has a water content of 5.0 to 8.0%.

[23] A capsule stable against mastication which is easily disintegrated in the stomach, comprises an oral irritative compound, has a capsule shell comprising (a) at least one capsule base selected from a protein, a saccharide, a biodegradable plastic and a hydrogenated fat and (b) a plasticizer, and has at least one property selected from the following (A) to (F):

(A) wherein it has a strength of 150 to 400 N by a cracking test;
(B) wherein it has a disintegration time of 3 to 10 minutes by the disintegration test stipulated in Japanese Pharmacopoeia;
(C) wherein the capsule shell has a shell thickness of 0.05 to 0.50 mm;
(D) wherein the capsule shell has a first seam thickness of 0.10 to 0.55 mm;
(E) wherein the capsule shell has a second seam thickness of 0.05 to 0.50 mm;
(F) wherein the capsule shell has a water content of 5.0 to 9.0%.

[24] A method for stabilizing a soft capsule against mastication, which comprises providing a soft capsule comprising an oral irritative compound and having a capsule shell comprising (a) at least one capsule base selected from a protein, a saccharide, a biodegradable plastic and a hydrogenated fat and (b) a plasticizer, with at least one property selected from the following (A) to (E):

(A) wherein it has a strength of 150 to 400 N by a cracking test;
(B) wherein the capsule shell has a shell thickness of 0.05 to 0.50 mm;
(C) wherein the capsule shell has a first seam thickness of 0.10 to 0.55 mm;
(D) wherein the capsule shell has a second seam thickness of 0.05 to 0.50 mm;
(E) wherein the capsule shell has a water content of 5.0 to 9.0%.

[25] A fast-dissolving soft capsule comprising (2R)-2-propyloctanoic acid or a salt thereof,
[26] The capsule according to [25], which has a dissolution rate of 90% or more 30 minutes after commencement of the dissolution test according to the second dissolution test stipulated in Japanese Pharmacopoeia;
[27] The capsule according to [25], wherein dissolution delay accompanied by preservation is reduced;
[28] The capsule according to [27], which has a dissolution rate of 90% or more 30 minutes after commencement of the dissolution test according to the second dissolution test stipulated in Japanese Pharmacopoeia, after preservation at room temperature for about one and half years;
[29] A fast-dissolving soft capsule comprising (2R)-2-propyloctanoic acid, which has a capsule shell comprising (a) gelatin and (b) glycerin which is contained in an amount of 30 parts by mass relative to 100 parts by mass of the gelatin, and has a dissolution rate of 90% or more 30 minutes after commencement of the dissolution test according to the second dissolution test stipulated in Japanese Pharmacopoeia, after preservation at room temperature for about one and half years;
[30] The capsule according to [29], which comprises 50 to 400 mg of (2R)-2-propyloctanoic acid;
[31] A method for reducing a dissolution delay accompanied by preservation of a soft capsule comprising (2R)-2-propyloctanoic acid and having a capsule shell comprising a gelatin, a glycerin and a sorbitol, which comprises adjusting a content of sorbitol in the capsule shell of said soft capsule to 20 parts by mass relative to 100 parts by mass of the gelatin.

In the present invention, (2R)-2-propyloctanoic acid is a compound represented by formula (I):

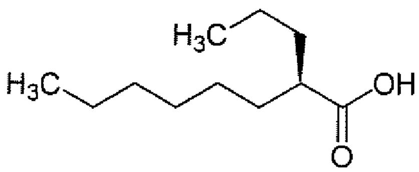

(I)

wherein 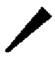 indicates that it is β configuration.

In the present invention, it is desirable that the salt of (2R)-2-propyloctanoic acid is a pharmacologically acceptable salt. It is desirable that the pharmacologically acceptable salt has no toxicity and is soluble in water. The appropriate salt of (2R)-2-propyloctanoic acid includes, for example, a salt with an inorganic base, a salt with an organic base, a salt with a basic natural amino acid and the like. As the salt with an inorganic base, for example, an alkali metal salt (e.g., sodium salt, potassium salt, lithium salt, *etc.),* an ammonium salt (e.g., tetramethylammonium salt, tetrabutylammonium salt, *etc.)* and the like are preferable. As the salt with an organic base, for example, salts with alkylamine (e.g., methylamine, dimethylamine, trimethylamine, triethylamine, *etc.*), heterocyclic amine (e.g., pyridine, picoline, piperidine, *etc.*), alkanol amine (e.g., ethanolamine, diethanolamine, triethanolamine, *etc.*), dicyclohexylamine, N,N'-dibenzylethylenediamine, cyclopentylamine, benzylamine, phenethylamine, tris(hydroxymethyl)methylamine, N-methyl-D-glucamine and the like are preferable. The salt with a basic natural amino acid is not particularly limited, so long as it is a salt with a basic amino acid which is present in the nature and can be purified, but, for example, a salt with arginine, lysine, ornithine, histidine or the like is preferable. Among these salts, preferred are, for example, an alkali metal salt, a basic natural amino acid salt and the like, and a sodium salt is particularly preferable.

The (2R)-2-propyloctanoic acid or a salt thereof can be produced in accordance with a conventionally known method such as the method described in EP0632008, WO99/58513, WO00/48982, Japanese Patent No. 3032447, Japanese Patent No. 3084354, WO03/051852, WO04/110972 or the like, or the method described in, for example, Comprehensive Organic Transformants: A Guide to Functional Group Preparations, 2nd Edition (Richard C. Larock, John Wiley & Sons Inc, 1999) or the like, or by optionally combining these methods. The reaction product can be purified by general purification means such as distillation under ordinary pressure or a reduced pressure, a high performance liquid chromatography, thin layer chromatography or column chromatography, which uses silica gel or magnesium silicate, washing, recrystallization and the like. In addition, it may be subjected to a treatment such as freeze drying, if

necessary.

The (2R)-2-propyloctanoic acid or a salt thereof is not particularly limited, so long as it is substantially pure and single substance, so that it may contain impurities (e.g., by-products, solvents, raw materials and the like which are derived from the production process, or degradation products thereof, *etc.)* within such a range that it is acceptable as a medicinal bulk. The content of impurities acceptable as the medicinal bulk varies depending on whether the (2R)-2-propyloctanoic acid is used or a salt thereof is used, and also varies depending on the contained impurities, but in the case of the (2R)-2-propyloctanoic acid, for example, it is desirable that heavy metals are about 20 ppm or less, the S-form as its optical isomer is about 1.49% by mass or less, 2-propanol and heptane as the residual solvents are about 5000 ppm or less in total, and the moisture is about 0.2% by mass or less. Particularly, (2R)-2-propyloctanoic acid having an optical purity of about 99% e.e. or more, more particularly, (2R)-2-propyloctanoic acid having an optical purity of about 99.3% e.e. or more, is suitable.

In the present invention, the (2R)-2-propyloctanoic acid or a salt thereof may be in any form. For example, it may be a liquid or a solid, or may be a semisolid having appropriate fluidity (e.g., solid such as gel-like or wax-like, *etc.).* Such a solid can be used with no particular limitation, even when it is a crystal or a non-crystal (amorphous) or a mixture thereof at an optional ratio. Specifically, it may be a liquid such as (2R)-2-propyloctanoic acid or a wax-like solid such as (2R)-2-propyloctanoic acid sodium salt.

In the present invention, preferred as the (2R)-2-propyloctanoic acid or a salt thereof are (2R)-2-propyloctanoic acid and (2R)-2-propyloctanoic acid sodium salt. Particularly, (2R)-2-propyloctanoic acid is desirable.

In the present invention, the capsule stable against mastication means a capsule from which the contents are not easily leaked at the time of mastication. Although intentional mastication and unintentional mastication are included in the mastication, the capsule stable against mastication of the present invention can show the effect of not easily leaking the contents, more effectively when the mastication is an unintentional mastication.

In the present invention, the capsule may be a substance which is generally called capsule by those skilled in the art. For example, as is described in detail in the following, it may be a soft capsule or a hard capsule. In the present invention, a soft capsule is preferable. The following discloses the present invention using a soft capsule as an example.

The soft capsule stable against mastication comprising (2R)-2-propyloctanoic acid or a salt thereof disclosed by the present invention (hereinafter sometimes referred to as the capsule of the present invention) has a property of (1) having a capsule shell comprising (a) at least one capsule base selected from a protein, a saccharide, a biodegradable plastic and a hydrogenated fat and (b) a plasticizer, and having a water content of 4.0 to 10.0%; and/or (2) being easily disintegrated in the stomach, preferably has both of these characteristics.

In this case, the property (1) of the present invention relates to the composition of capsule shell. Similar to the case of general soft capsule, the capsule of the present invention is constituted from an inclusion liquid and a capsule shell. The capsule shell contains (a) a capsule base as a substance which becomes the main body of the capsule shell and (b) a plasticizer, and if desired, for example, a perfume (e.g., mentha oil, cinnamon oil, fruit essence such as strawberry, flavor, *etc.*), an antiseptic (e.g., ethyl parahydroxybenzoate, propyl parahydroxybenzoate, *etc.*), a coloring matter (e.g., Yellow No. 4, Yellow No. 5, Red No. 3, Blue No. 1, copper chlorophylline, *etc.),* an opaquer (e.g., titanium dioxide, red iron oxide, iron sesquioxide, *etc.*), a solubility adjusting agent (e.g., cellulose acetate phthalate, an alkali metal salt of hydroxypropylmethylcellulose, an alkali metal salt of hydroxymethylcellulose acetate succinate, an alginic acid alkali salt, a polyacrylic acid alkali metal salt, methyl cellulose, carboxymethylcellulose, casein, collagen, agar powder, polyvinyl alcohol, pectin, *etc.*) and the like can also be added thereto. In this specification, such a capsule shell also contains water as its composing component similar to the case of the capsule shell generally used in the soft capsule. The water content in the capsule shell is expressed using an index of water content as is described later.

The capsule base to be used in the capsule shell of the present invention may be any substance which can be used as the base of soft capsule shell. For example, proteins (e.g., gelatin, hydrolyzed gelatin, collagen, hydrolyzed collagen, casein, *etc.*), saccharides (e.g., starch, amylose, polygalacturonic acid, agar, carrageenan, gum arabic, gellan gum, xanthan gum, pectin, alginic acid, *etc.*), biodegradable plastics (e.g., polylactic acid, polyhydroxybutyric acid, polyglutamic acid, *etc.*), hydrogenated fats (e.g., triglycerides and diglycerides of middle-chain fatty acids (e.g., butter, margarine, shortening, cacao butter, *etc.), etc.)* and the like can be suitably used. These capsule bases can also be used in combination. In the present invention, a protein, particularly gelatin, is preferable among these capsule bases. This gelatin may be any gelatin which can be used as the base of soft capsule shell. For example, in addition to those which are generally called gelatin, this may be a alkali treated gelatin, an acid treated gelatin, a peptide gelatin, a low molecular gelatin, a gelatin derivative, a chemically modified gelatin, a succinated gelatin or the like. In addition, regarding its origin, the gelatin derived from various animals such as mammals (e.g., cattle, pig, *etc.),* fishes (e.g., tilapia, sea bream, tuna, catfish, *etc.*), birds (e.g., domestic fowl, ostrich, *etc.*) and the like can be used without particular limitation.

The plasticizer to be used in the capsule shell of the present invention may be any substance which can be used as the plasticizer of the soft capsule shell. For example, a sugar (e.g., sucrose, white sugar, starch syrup, *etc.),* a sugar alcohol (e.g., sorbitol, xylitol, mannitol, *etc.),* a polyhydric alcohol (e.g., glycerine, ethylene glycol, polyethylene glycol, propylene glycol, *etc.*) and the like can be suitably used. More preferred are, for example, glycerine, sorbitol,

polyethylene glycol and the like, and glycerine or sorbitol, particularly glycerine, is preferable. This glycerine may be any substance which is generally called glycerine, but for example, official glycerine and concentrated glycerine are preferable, and, for example, concentrated glycerine is particularly preferable.

As described above, a capsule shell which uses gelatin as the capsule base is desirable as the capsule shell in the capsule of the present invention. A capsule shell which uses gelatin as the capsule base and contains glycerine as the plasticizer is particularly desirable. Such a capsule shell may further contain sorbitol as a plasticizer, if necessary, and it is desirable that the sorbitol content in that case is about 20 parts by mass or less relative to 100 parts by mass of gelatin as the capsule base. In addition, when sorbitol is contained, it is desirable that the glycerine content is also about 20 parts by mass or less relative to 100 parts by mass of gelatin as the capsule base. On the other hand, when sorbitol is not substantially contained, mixing ratio of the capsule base (particularly gelatin) and glycerine is preferably from about 25 to about 40 parts by mass of glycerine relative to 100 parts by mass of the capsule base, particularly, about 30 parts by mass of glycerine relative to 100 parts by mass of the capsule base is preferable.

The property (2) of the present invention relates to the disintegrating property of the capsule. In order to add such a property to the capsule of the present invention, composition of the capsule shell may be adjusted as described above using disintegration time or the like of such a capsule as the index, or thickness of the capsule shell, water content of the capsule shell or the like may be adjusted as described later. The disintegration time of capsule necessary for providing the capsule of the present invention with the above-described property of being "easily disintegrated in the stomach" is from about 3 to about 10 minutes, more preferably from about 5 to about 8 minutes, particularly preferably from about 5.7 to about 6.3 minutes, by a disintegration test. The disintegration test can be carried out by a conventionally known method. For example, it is desirable to carry out this by a method generally known as a disintegration test of capsule, particularly the disintegration test described in the pharmacopoeia of each country, more particularly the disintegration test described in Japanese Pharmacopoeia, most particularly the disintegration test described in the 14th edition of Japanese Pharmacopoeia. All of the soft capsules which contain (2R)-2-propyloctanoic acid or a salt thereof and show the above-described disintegration time by the disintegration test described in Japanese Pharmacopoeia are included in the present invention, regardless of the values shown by any other disintegration test.

In addition, it is desirable that the property (2) of the present invention, namely the property of being "easily disintegrated in the stomach", does not cause a periodical change accompanied by the preservation of the capsule. It is known in general that a soft capsule causes delay of dissolution accompanied by its preservation, depending on the component of inclusion liquid and the capsule shell component. The dissolution delay means that a certain change occurs periodically in the capsule shell when a soft capsule is preserved, and disintegration property and dissolution property of said capsule become worsen thereby. That is, since quick disintegration and dissolution at the time of internal taking are not effected in the case of a soft capsule which caused dissolution delay, desirable drug effect cannot be obtained in some cases. Accordingly, it is desirable that the property (2) of the present invention, namely the property of being "easily disintegrated in the stomach", does not cause a periodical change or hardly causes the same, does not cause the so-called dissolution delay or hardly causes the same.

Degree of the "dissolution delay" can be verified by subjecting the capsule of the present invention to a dissolution test after its optional period of preservation, and comparing the result with the initial value. The dissolution test can be carried out a conventionally known method. For example, it is desirable to carry out this by a method generally known as a dissolution test of solid pharmaceutical preparations for internal use such as tablets and capsules, particularly the dissolution test described in the pharmacopoeia of each country, more particularly the dissolution test described in Japanese Pharmacopoeia, most particularly the dissolution test described in the 14th edition of Japanese Pharmacopoeia. Specifically, it is desirable to carry out this by the paddle method of the second dissolution test of Japanese Pharmacopoeia, as is described later in Examples. In this specification, the initial value means a value obtained by subjecting said capsule to the above-described dissolution test at the time of substantially just after its production (e.g., from just after the production to 10 days or less after the production or the like, preferably from just after the production to 5 days or less after the production or the like, more preferably from just after the production to 3 days or less after the production or the like, particularly preferably from just after the production to 24 hours or less after the production, *etc.*). The preservation condition for verifying the degree of "dissolution delay" is not particularly limited, but, for example, it may be room temperature as generally carried out by those skilled in the art, or may be a condition of high temperature and/or high humidity as is generally called severe test. By setting to conditions at high temperature and/or high humidity, the result of long-term preservation at room temperature can be obtained within more shorter period.

In the present invention, the "dissolution delay was reduced" means that the dissolution rate after optional period of preservation is not substantially changed in comparison with the initial value (dissolution rate), or the rate of change in dissolution rate is within 20%, preferably within 15%, more preferably within 10%. In this case, the dissolution rate can be calculated by the above-described dissolution test. Specifically, when amount of a drug in one capsule used in the dissolution test is defined as 100, amount of the drug eluted in the test liquid after a lapse of optional period of time starting from the commencement of the dissolution test can be regarded as the dissolution ratio. For example, the "dissolution rate 30 minutes after commencement of the test" means a dissolution rate when the dissolution test was

carried out for 30 minutes. Regarding the measurement of the amount of eluted drug, it can be obtained by measuring absorbance at an ultraviolet region, and can be obtained, for example, by a high performance liquid chromatography or the like. In addition, the rate of change (%) can be calculated by the following formula:

$$\text{Rate of change (\%)} = ((A - B)/A) \times 100$$

(A: initial value (dissolution rate); B: dissolution rate after an optional period of preservation).

In this specification, although it is a matter of course to those skilled in the art, it is necessary that the condition of dissolution test for obtaining the rate of change (%) is identical between the sample before the optional period of preservation and the sample after the preservation. For example, when the rate of change is obtained using the dissolution rate 30 minutes after commencement of the test, in the dissolution test after preservation of an optional period of time, it is necessary to use the value of a dissolution rate 30 minutes after commencement of the dissolution test carried out under the same condition, also on the initial value (dissolution rate).

Although the preservation condition and length of period for verifying the presence or absence of the dissolution delay are not particularly limited, a condition which reflects its preservation as a medicine at the hospital, pharmacy, patient's home and the like is desirable. Specifically, preservation at room temperature can be exemplified, and preservation at from about 25 to about 30°C for about 1.5 years (e.g., from about 16 to about 20 months or the like, preferably from about 17 to about 19 months, *etc.*) can be exemplified as is described later in Examples as an index thereof

In the present invention, the fast-dissolving soft capsule means a soft capsule which can release the inclusion liquid in said capsule through fast-dissolving of the shell after internal taking thereof. Degree of the "quick dissolution" of the fast-dissolving soft capsule can be proved by various dissolution test methods such as the methods described in the pharmacopoeias as described above (e.g., the paddle method of the second dissolution tests of Japanese Pharmacopoeia, as is described later in Examples) and the like.

In the present invention, the fast-dissolving soft capsule which comprises (2R)-2-propyloctanoic acid or a salt thereof may be any soft capsule which contains (2R)-2-propyloctanoic acid or a salt thereof and shows the above-described fast-dissolving property. Specifically, a soft capsule having a dissolution rate, 30 minutes after commencement of the test by the above-described dissolution test, of about 60% or more (about 60 to about 100%), preferably about 80% or more (about 80 to about 104%), more preferably about 90% or more (about 90 to about 100%), and the like can be cited. Every soft capsule which comprises (2R)-2-propyloctanoic acid or a salt thereof and satisfies this parameter is included in the fast-dissolving soft capsule of the present invention. The dissolution test may be carried out any time after production of the soft capsule, but, for example, it is desirable to measure it at the time of substantially just after the production as described above. By carrying out the dissolution test at the time of substantially just after the production, whether or not said soft capsules were produced as the fast-dissolving soft capsules can be judged accurately.

According to the soft capsule stable against mastication disclosed by the present invention, the property as the mastication stabilization relates to the strength, namely hardness, of the capsule. In order to provide the capsule of the present invention with such a property, thickness of the capsule shell, water content of the capsule shell and the like may be adjusted. Strength of the capsule suitable for providing the capsule of the present invention with stability against mastication, namely the above-described property of "the contents are not easily leaked at the time of mastication", is preferably about 150 newton (hereinafter referred to as "N") or more, more preferably from about 150 to about 400 N, particularly preferably from about 180 to about 350 N, most particularly preferably from about 193 to about 310 N, by a cracking test. The cracking test can be carried out by a conventionally known method. Specifically, as is shown later in Examples, for example, it can be measured using a pressurization machine which can compress a sample from upside and downside with two parallel planes, such as a compact table-top univarsal tester manufactured by Shimadzu Corp. (EZ Test-500N).

As described above, the capsule shell of the capsule of the present invention contains water in addition to the capsule base and plasticizer, and strength of the capsule of the present invention can be adjusted by adjusting the amount of water contained in the capsule shell, namely water content. The water content of capsule shell suitable for providing the capsule of the present invention with the above-described strength is from about 5.0 to about 9.0%, more preferably from about 5.0 to about 8.0%, particularly preferably from about 5.6 to about 7.3%. The water content of capsule shell can be measured by a conventionally known method. Specifically, as is shown later in Examples, for example, it can be calculated based on the following formula,

$$\text{Water content (\%)} = ((\text{weight before drying - weight after drying})/\text{weight before drying}) \times 100$$

by using a capsule shell as the sample and measuring its weight (weight before drying) and its weight (weight after drying) after drying the sample at a high temperature (e.g., 105°C, *etc.).*

The thickness of capsule shell suitable for providing the capsule of the present invention with the above-described strength varies depending on which thickness among the shell thickness, the first seam thickness and the second seam thickness is measured. In this case, the "shell thickness", "first seam thickness" and "second seam thickness" are terms generally used by those skilled in the art, and the "shell thickness" means a thickness of a part where the shell swells most largely by the charging of inclusion liquid when the capsule is formed, the "first seam thickness" is a thickness of a part where a sheet for capsule shell is firstly joined when the capsule is formed, and the "second seam thickness" is a thickness of a part where the sheet for capsule shell is lastly joined when the capsule is formed. In this specification, the sheet for capsule shell means a sheet which contains components of the capsule shell and is used in a soft capsule production method generally called stamping method, such as the rotary die method. By the rotary die method, a soft capsule of an optional shape such as oval type, round type, suppository type, oblong type, tube type or the like can be produced by charging the inclusion liquid while forming two sheets for capsule shell using a die roll which corresponds to each shape of the soft capsule.

Thickness of each part of the capsule shell can be measured by a conventionally known method. For example, it can be effected by cutting central part of the soft capsule in a round slice using a sharp edged tool such as a pair of scissors or a knife, washing the inclusion liquid using an organic solvent (e.g., n-hexane, ether, acetone, hydrochlorofluorocarbon, *etc.*), and then measuring the cut surface using a scale or the like while observing it under an optical microscope. The thickness of capsule shell suitable for providing the capsule of the present invention with the above-described strength is preferably from about 0.05 to about 0.50 mm, more preferably from about 0.10 to about 0.45 mm, particularly preferably from about 0.14 to about 0.37 mm, as the shell thickness, preferably from about 0.10 to about 0.55 mm, more preferably from about 0.15 to about 0.50 mm, particularly preferably from about 0.18 to about 0.42 mm, as the first seam thickness, and preferably from about 0.05 to about 0.50 mm, more preferably from about 0.10 to about 0.40 mm, particularly preferably from about 0.14 to about 0.36 mm, as the second seam thickness. Strength of the capsule of the present invention can be further improved by controlling any of shell thickness, the first seam thickness or the second seam thickness, preferably all of the thickness, of the capsule shell, within the above-described ranges.

Thus, when the capsule is formed in such a manner that the thickness of capsule shell and the water content of capsule shell are respectively set within the above-described ranges, preferably both within the above-described ranges, the capsule of the present invention can be provided with the above-described strength, and the capsule of the present invention can be provided with the above-described property of being "the contents are not easily leaked at the time of mastication", so that it can be provided as a capsule stable against mastication.

In the present invention, a soft capsule or the like, as a soft capsule which comprises (2R)-2-propyloctanoic acid and contains (a) gelatin and (b) about 30 parts by mass of glycerine relative to 100 parts by mass of gelatin, wherein it has a capsule shell having a water content of about 5.0 to about 8.0%, a shell thickness of about 0.10 to about 0.45 mm, a first seam thickness of about 0.15 to about 0.50 mm and a second seam thickness of about 0.10 to about 0.40 mm, and shows a strength of about 180 to about 350 N by a cracking test, can be exemplified as one of the suitable embodiments of the capsule of the present invention having both of the above-described characteristics of (1) and (2). Since such a soft capsule shows a disintegration time of about 5 to about 8 minutes by the disintegration test stipulated in Japanese Pharmacopoeia, this is a soft capsule which has the characteristics of not easily leaking the contents at the time of mastication and easily disintegrating in the stomach and satisfies all of the problems of the present invention.

The capsule of the present invention can be produced in accordance with a conventionally known soft capsule production method. Specifically, it can be produced by preparing (1) an inclusion liquid comprising (2R)-2-propyloctanoic acid or a salt thereof and (2) a solution for capsule shell, subjecting them, for example, to a conventionally known encapsulating method such as a stamping method (e.g., a rotary die method, *etc.)* in such a manner that the thickness of the capsule shell becomes the above-described range, the water content of the capsule shell also becomes the above-described range, and the disintegration time, strength and the like also become the above-described ranges, and then drying the thus obtained capsule.

The solution for capsule shell can be used without particular limitation, so long as it contains components which become the above-described capsule shell composition, can form a thin shell (corresponds to the above-described sheet for capsule shell) under a molten state or dissolved state and is solidified when cooled and/or dried after formation of the shell. Preferably, such a solution for capsule shell can be prepared by mixing the above-described capsule base and plasticizer with an appropriate amount of water (e.g., purified water, *etc.).* If necessary, a heating operation may be carried out in the solution for capsule shell preparation process.

In the present invention, the inclusion liquid comprising (2R)-2-propyloctanoic acid or a salt thereof can be prepared using (2R)-2-propyloctanoic acid or a salt thereof. The inclusion liquid may have an appropriate fluidity (e.g., a fluidity which can be injected using a soft capsule encapsulation machine (e.g., a fluidity having a viscosity of about 50000 millipascal second (mPa.s) or less, *etc.),* or the like), and it may be a liquid as a matter of course but may also be a semisolid or the like that shows a gel-like form. The inclusion liquid preparation method can be optionally changed

depending on whether such a (2R)-2-propyloctanoic acid or a salt thereof is a solid, liquid or semisolid.

When the (2R)-2-propyloctanoic acid or a salt thereof is a liquid, it may be used directly as the inclusion liquid or used by adding an appropriate additive agent, if necessary. Also, it may be used by mixing with fats or the like. The fats include, for example, medium-chain fatty acids [for example, plant oil (e.g., soybean oil, cotton seed oil, safflower oil, corn oil, olive oil, coconut oil, *Perilla* oil, oil of *Perilla frutescens* var *frutescens, etc.),* fish oil (e.g., cod liver oil, *etc.),* and the like], middle-chain fatty acid triglycerides (medium-chain triglycerides: MCT) [e.g., Panacet (trade name, manufactured by Nippon Oil & Fats), ODO (trade name, manufactured by Nisshin Oil Mils) and the like], and chemically synthesized triglycerides [e.g., triglycerides of known compositions, lipids of known structures and the like, such as 2-linoleoyl-1,3-dioctanoyl glycerol (8L8), 2-linoleoyl-1,3-dididecanoyl glycerol (10L10)], and the like. These fats may be used alone or as a combination of two or more.

As the additive agent, additive agents generally used in oral administration preparations can be used with no particular limitation. Particularly, additive agents which are used in soft capsule preparations and oral solutions (e.g., an antiseptic, a preservative, a surfactant, a solubilizer, an emulsifier, a solvent, a pH adjusting agent, a buffer agent, a suspending agent, a thickening agent, a stabilizing agent, a solubilizing aid, *etc.)* are suitably used. Two or more of these additive agents may be used in combination if necessary. The antiseptic and preservative include, for example, benzoic acid, sodium benzoate, sodium sorbate, parabens (e.g., ethyl parahydroxybenzoate, butyl parahydroxybenzoate, propyl parahydroxybenzoate, *etc.)* and the like. The surfactant, solubilizer, emulsifier and solvent include, for example, hydroxypropylmethylcellulose, polyvinyl pyrrolidone, sucrose fatty acid ester, polyoxyethylene hydrogenated castor oils, polysorbate 80, polyethylene glycol, glycerine, ethanol, propylene glycol, water (e.g., purified water, distilled water for injection, *etc.*) and the like. The pH adjusting agent and buffer agent include, for example, an inorganic acid or a base such as alkali or the like, for example, hydrochloric acid, sodium hydroxide, potassium hydroxide or sodium bicarbonate, and also an organic acid such as citric acid, malic acid, tartaric acid, or succinic acid or a salt thereof, and the like. The pH can be adjusted by a pH-adjusting method generally used in the technical field of oral solutions or in accordance with a method thereof The suspending agent and thickening agent include, for example, gum arabic, crystalline cellulose, bee gum, xanthan gum, gelatin, metolose and an edible salt thereof, carmellose and an edible salt thereof, and the like. The stabilizing agent includes, for example, an edible salt of edetic acid, sodium chloride, an edible salt of pyrosulfurous acid, and the like. The solubilizing aid includes, for example, cyclodextrin, arginine, and the like. These additive agents are mixed at a ratio generally employed in oral administration preparations. Also, in addition to the above-described substances, the additive agents described, for example, in a conventionally known reference such as "Iyakuhin Tenkabuthu Jiten (Dictionary of Pharmaceutical Additives) (edited by Japan Society of Pharmaceutical Additives) published in 2000 by Yakuji Nippo may be used.

On the other hand, the (2R)-2-propyloctanoic acid or a salt thereof is a solid, it can be used as the inclusion liquid by dissolving, suspending or emulsifying it in an appropriate solvent (for example, water (e.g., distilled water for injection, purified water, *etc.),* the above-described middle-chain fatty acid, the above-described middle-chain fatty acid triglyceride, or the like) in the presence or absence of an appropriate additive agent. The additive agent includes the above-described additive agents generally used in oral administration preparations and the like. In addition, these additive agents can be added by combining two or more components, if necessary.

In addition, when the (2R)-2-propyloctanoic acid or a salt thereof is a semisolid, it may be directly used as the inclusion liquid similar to the case of liquid, or may be used by adding an appropriate additive agent, if necessary. Also, similar to the case of solid, it can also be used as the inclusion liquid by dissolving, suspending or emulsifying it in a appropriate solvent in the presence or absence of an appropriate additive agent. As the additive agent and solvent to be used in making a semisolid into the inclusion liquid, the same substances described in the above may be used.

An inclusion liquid which comprises (2R)-2-propyloctanoic acid can be exemplified as a preferable inclusion liquid of the present invention. As described above, (2R)-2-propyloctanoic acid is liquid, so that it is particularly desirable to use it directly as the inclusion liquid. In addition, for example, it is also desirable to use it by mixing with the above-described fats.

Also, since (2R)-2-propyloctanoic acid is liquid (oil), it can be used as an aqueous inclusion liquid by adding an appropriate additive agent. The additive agent suitable for using it as an aqueous inclusion liquid includes, for example, a metal compound which shows basicity in an aqueous solution, and the like. The metal compound which shows basicity in an aqueous solution is not particularly limited, but examples include metal salts (for example, monovalent alkali metal salts (e.g., sodium salt, potassium salt, lithium salt, rubidium salt, cesium salt, francium salt, *etc.),* or the like) of weak acids (for example, phosphoric acid, carbonic acid, boric acid, sulfurous acid, organic sulfonic acid, organic carboxylic acid having from 2 to 6 carbon atoms (e.g., mono- to trivalent organic carboxylic acid having from 2 to 6 carbon atoms, namely aliphatic monocarboxylic acid, dicarboxylic acid or tricarboxylic acid having from 2 to 6 carbon atoms, *etc.*), other organic acids, or the like) and metal hydroxides (for example, monovalent alkali metal hydroxides (e.g., sodium hydroxide, potassium hydroxide, lithium hydroxide, rubidium hydroxide, cesium hydroxide, francium hydroxide, *etc.*), or the like). These metal compounds may be used by combining two or more components, if necessary. The (2R)-2-propyloctanoic acid can be used as an aqueous inclusion liquid in the capsule of the present invention, by mixing with these metal

compounds, and further mixing with water, if necessary.

As the drying method of capsule, it may be any method which can dry such a capsule while maintaining its shape, but a method in which two steps of tumbler drying and tray drying are used in combination is preferably used. By carrying out these two steps of drying steps, uniform soft capsule of the present invention having a regular shape can be produced. In this specification, the capsule before subjecting to such a drying process is sometimes called "capsule before drying" herein, in order to distinguish it from the capsule after drying.

The tumbler drying is carried out at from about 15 to about 40°C, preferably from about 20 to about 35°C, particularly preferably from about 23 to about 30°C, for from several hours to several days, preferably from about 0.5 hour to about 1 day, more preferably from about 1 to about 12 hours, particularly preferably from about 1.5 to about 6 hours.

The tray drying is carried out at from about 10 to about 40°C, preferably from about 15 to about 35°C, particularly preferably from about 20 to about 30°C, for from several hours to several days, preferably from about 6 hours to about 4 days, more preferably from about 12 hours to about 3 days, particularly preferably from about 1 to about 2 days.

In the present invention, an amount of the (2R)-2-propyloctanoic acid or a salt thereof to be contained per one capsule is not particularly limited. Preferred is a soft capsule which comprises (2R)-2-propyloctanoic acid or a salt thereof in an amount of about 30 to about 600 mg, more preferably from about 50 to about 400 mg, particularly preferably from about 100 to about 300 mg, per 1 capsule. Particularly suitable is a soft capsule which comprises about 100 mg or about 300 mg per capsule of (2R)-2-propyloctanoic acid or a salt thereof.

When produced in the above-described manner, an optional packaging is applied to the capsule of the present invention, if necessary. Such a packaging may, for example, be a not individually packaged non-unit packaging (e.g., a bulk packaging) or the like, but, for example, a hermetically sealed packaging such as heat sealing, in which a medicine is sealed with a thermo-adhesive film, is desirable. In this specification, for example, the packaging using a "airtight container" or "hermetically sealed container" as provided by the Pharmacopoeia is also included as its meaning in the hermetically sealed packaging of the present invention. PTP (press through pack) packaging, SP (strip package) packaging and the like are included in the heat sealing, and PTP packaging is particularly desirable. The material for the PTP packaging includes PVC (polyvinyl chloride), PVDC (polyvinylidene chloride) coat PVC (polyvinyl chloride), PP (polypropylene), polypropylene system multilayer, and the like. It is desirable that the PTP packaging is used jointly with an aluminum packaging. In addition, after carrying out the PTP packaging or SP packaging, a certain quantity of the capsule of the present invention may be subjected to a secondary packaging with polyethylene or aluminum foil (so-called pillow packaging).

The characteristics of "easy disintegration in the stomach" and "not easily leaking the contents at the time of mastication possessed by the capsule of the present invention are also applicable when an oral irritative compound is used as the inclusion liquid, instead of the (2R)-2-propyloctanoic acid or a salt thereof

In this specification, the oral irritative compound is not particularly limited, so long as it is a compound which causes an unpleasant taste (e.g., acridness, pain, bitterness, burning sensation, *etc.)* when such a compound is taken into the oral cavity. Whether or not it causes an unpleasant taste in the oral cavity can be easily judged by carrying out a conventionally known test which is generally called sensory test. The oral irritative compound includes, for example, L-tryptophan, L-methionine, L-lysine, L-leucine, azithromycin, aminopyrine, aminophylline, erythromycin, casein, caffeine, clarithromycin, chloramphenicol, gentian, saponin, digitoxin, cyclandelate, sulpyrine, cefalexin, *Swertia japonica,* tannin, tetracycline, calcium pantothenate, phenobarbital, oxapium iodide, limonene, rescinnamine, potassium chloride, berberine chloride, azelastine hydrochloride, aminoguanidine hydrochloride, etilefrine hydrochloride, quinine hydrochloride, diltiazem hydrochloride, cefotiam hexetyl hydrochloride, cefcanel daloxate hydrochloride, talampicillin hydrochloride, ticlopidine hydrochloride, donepezil hydrochloride, bacampicillin hydrochloride, papaverine hydrochloride, hydralazine hydrochloride, propranolol hydrochloride, promethazine hydrochloride, meclofenoxate hydrochloride, lenampicillin hydrochloride, dextromethorphan hydrobromide, quinine sulfate, mafnesium sulfate, a class of vitamin B (e.g., thiamine, riboflavin, pyridoxine, cyanocobalamin or derivatives thereof, *etc.),* and the like.

Encapsulation of the oral irritative compound into the capsule can be carried out in accordance with the case of the above-described (2R)-2-propyloctanoic acid or a salt thereof Similar to the case of the above-described soft capsule which comprises (2R)-2-propyloctanoic acid or a salt thereof, the soft capsule that comprises an oral irritative compound as the active ingredient, produced by the same method as described above, is a soft capsule which is easily disintegrated in the stomach and is stable to mastication, namely the contents are not easily leaked at the time of mastication.

On the other hand, as described above, characteristics of the capsule of the present invention to be provided in the form of soft capsule, namely the characteristics of "easy disintegration in the stomach" and "not easily leaking the contents at the time of mastication" are also applicable to a case in which the (2R)-2-propyloctanoic acid or a salt thereof is encapsulated in a hard capsule.

The hard capsule generally consists of two parts of the cap and the body and is provided in such a form that a medicinal component is encapsulated therein. In order to provide such a hard capsule with the characteristics of "easy disintegration in the stomach" and "not easily leaking the contents at the time of mastication", such a hard capsule may

be produced in such a manner that it shows a strength of preferably about 150 newton (hereinafter referred to as N) or more, more preferably from about 150 to about 400 N, particularly preferably from about 180 to about 350 N, most particularly preferably from about 193 to about 310 N, by a cracking test, and also shows a disintegration time of about 3 to about 10 minutes, more preferably from about 5 to about 8 minutes, particularly preferably from about 5.7 to about 6.3 minutes, by a disintegration test, similar to the case of the above-described soft capsule.

Production of the hard capsule can be carried out in accordance with a conventionally known method (e.g., auger type, compress type, press type, disc type, *etc.*). In addition, when a liquid material is used as the inclusion substance, it is desirable to seal the junction separately, or to take such a measure that locking is effected at the time of inter-fitting, in order to prevent leaking of the liquid from the junction of the cap and body.

Application to Pharmaceuticals:

The capsule of the present invention, as it contains (2R)-2-propyloctanoic acid or a salt thereof as an active ingredient, are useful in prevention, treatment and/or suppression of, for example, neurodegenerative disease, neuropathy, or disease in need of nerve regeneration, or their progress, in a mammal (for example, human, non-human animals, e.g., monkey, sheep, bovine, equine, canine, feline, rabbit, rat, mouse, *etc.*).

The neurodegenerative disease includes all of diseases accompanied by degeneration of nerve cell, and is not limited by its cause. The neurodegenerative disease in the present invention also includes neuropathy or disease in need of nerve regeneration. The nerve cell may be any type of nerve cells in the living body, including, for example, central nerves (e.g., cerebral nerves, spinal nerves, *etc.*), peripheral nerves (e.g., autonomic nervous system (e.g., sympathetic nerve, parasympathetic nerve, *etc.*), *etc.)* and so on. The neurodegenerative disease is, for example, a disease of central nerve, including Parkinson's disease, Parkinson syndrome, Alzheimer's disease, Down's disease, amyotrophic lateral sclerosis, familial amyotrophic lateral sclerosis, progressive supranuclear palsy, Huntington's disease, spinocerebellar ataxia, dentatorubral-pallidoluysian atrophy, olivopontocerebellar atrophy, corticobasal degeneration, familial dementia, frontotemporal dementia, senile dementia, diffuse Lewy body disease, striatonigral degeneration, chorea athetosis, dystonia, Meiges's syndrome, late cortical cerebellar atrophy, familial spastic paraplegia, motor neuron disease, Machado-Joseph disease, Pick's disease, nervous dysfunction after cerebral apoplexy (for example, brain hemorrhage (e.g., hypertensive intracerebral hemorrhage, *etc.*), cerebral infarction (e.g., cerebral thrombosis, cerebral embolus, *etc.*), transient ischemic attack, subarachnoid hemorrhage, *etc.*), nervous dysfunction after cerebrospinal damage, demyelinating disease (for example, multiple sclerosis, Guillain-Barré syndrome, acute disseminated encephalomyelitis, acute cerebellitis, transverse myelitis, *etc.*), brain tumor (for example, astrocytoma, *etc.*), cerebrospinal disease caused by infection (for example, meningitis, cerebral abscess, Creutzfeldt-Jakob disease, AIDS dementia, *etc.*), mental disease (schizophrenia, manic-depressive psychosis, neurosis, psychosomatic disease, epilepsy, *etc.)* and the like. The neurodegenerative disease is more preferably Parkinson's disease, Parkinson syndrome, Alzheimer's disease, amyotrophic lateral sclerosis, familial amyotrophic lateral sclerosis, or the like.

The neuropathy includes all of the nervous dysfunction. That is, the neuropathy generally includes disorders recognized as a symptom during disease. For example, Parkinson's disease or Parkinson syndrome is accompanied by for example tremor, muscle rigidity (rigidity), bradykinegia, postural disturbance, dysautonomia, pulsion, gait disturbance, mental symptom, and so on. For example, Alzheimer disease is accompanied by dementia symptom; the amyotrophic lateral sclerosis or familial amyotrophic lateral sclerosis is accompanied by muscle atrophy, muscle weakness, dysfunction of upper extremity, gait disorder, dysarthria, dysphagia, respiration disorder, and so on.

The disease in need of nerve regeneration is means those in which the absolute number of the nerve cells are reduced by lack of nerve cells to spoil normal neural function, including, for example, the above neurodegenerative disease in such a state. In such a disease, a cell capable of generating normal nerves (for example, nerve stem cell, nerve precursor cell, nerve cell, other stem cell, glia cell, *etc.)* is transplanted surgically or an intrinsic cell of them is activated to regenerate the nerve for therapy. The capsule of the present invention may be administered temporarily or continuously at the time of transplantation of the above cell or of activation of the intrinsic cell to accelerate nerve regeneration.

Further, the capsule of the present invention is also useful as a nerve regeneration accelerator or S100β-increase inhibitor. The capsule of the present invention may be orally administrated to the living body with the aim of prevention, treatment and/or suppression of progress of the above-mentioned diseases. In the present invention, the term "prevention" means to prevent the onset of disease itself; "treatment" means to lead the state of disease to cure; and "suppression of progress" means to suppress the deterioration or stop the progress of the state.

Daily dose of the capsule of the present invention is not particularly limited, because it varies depending on the degree of symptoms; age, sex and body weight of the object to be administered; period and interval of the administration; kind of the active ingredient and the like, but, for example, when it is orally administered as a therapeutic agent for neurodegenerative disease such as Parkinson disease, Parkinson syndrome, Alzheimer disease, amyotrophic lateral sclerosis, or familial amyotrophic lateral sclerosis, and when it contains (2R)-2-propyloctanoic acid as the active ingredient, capsules containing from about 50 to about 5000 mg, more preferably from about 100 to about 1200 mg, of (2R)-2-propyloctanoic acid are administered as a daily dose.

When the capsule of the present invention is used, other medicament may used in combination. The other medicament which can be used with the capsule of the present invention in combination includes, for example, an anticonvulsant (e.g., phenobarbital, mephobarbital, metharbital, primidone, phenytoin, ethotoin, trimethadione, ethosuximide, acetylphenetride, carbamazepine, acetazolamide, diazepam, sodium valproate, *etc.),* an acetylcholinesterase inhibitor (e.g., donepezil hydrochloride, TAK-147, rivastigmine, galantamine, *etc.*), a neurotrophic factor (e.g., ABS-205, *etc.),* an aldose reductase inhibitor, an antithrombotic (e.g., t-PA, heparin), an oral anticoagulant (e.g., warfarin, *etc.*), a synthetic antithrombin drug (e.g., gabexate mesylate, nafamostat mesylate, argatroban, *etc.*), an antiplatelet drug (e.g., aspirin, dipyridamole, ticlopidine hydrochloride, beraprost sodium, cilostazol, sodium ozagrel, *etc.),* a thrombolytic agent (e.g., urokinase, tisokinase, alteprase, *etc.),* a Factor Xa inhibitor, a Factor VIIa inhibitor, a cerebral blood flow and metabolism improver (e.g., idebenone, calcium hopantenate, amantadine hydrochloride, meclofenoxate hydrochloride, dihydroergotoxine mesylate, pyrithioxin hydrochloride, γ-aminobutyric acid, bifemelane hydrochloride, lisuride maleate, indeloxazine hydrochloride, nicergoline, propentofylline, *etc.*)*,* an antioxidant (e.g., edaravone, *etc.),* a glycerin preparation (e.g., glyceol, *etc.*)*,* a β-secretase inhibitor (e.g., 6-(4-biphenylyl)methoxy-2-[2-(N, N-dimethylamino) ethyl] tetraline, 6-(4-biphenylyl)methoxy-2-(N, N-dimethylamino) methyltetraline, 6-(4-biphenylyl)methoxy-2-(N, N-dipropylamino)methyltetraline, 2-(N, N-dimethylamino) methyl-6-(4'-rnethaxybiphenyl-4-yl)methoxytetraline, 6-(4-biphenylyl)methoxy-2-[2-(N, N-diethylamino)ethyl] tetraline, 2-[2-(N, N-dimethylamino)ethyl]-6-(4'-methylbiphenyl-4-yl)methoxytetraline, 2-[2-(N, N-dimethylamino)ethyl]-6-(4'-methoxybiphenyl-4-yl)methoxytetraline, 6-(2',4'-dimethoxybiphenyl-4-yl)methoxy-2-[2-(N, N-dimethylamino)ethyl] tetraline, 6-[4-(1,3-benzodioxol-5-yl)phenyl] methoxy-2-[2-(N, N-dimethylamino) ethyl] tetraline, 6-(3',4'-dimethoxybiphenyl-4-yl)methoxy-2-[2-(N, N-dimethylamino)ethyl] tetraline, and optical isomers, salts and hydrates thereof, OM99-2 (WO 01/00663), *etc.*)*,* β-amyloid protein aggregation inhibitor (e.g., PTI-00703, ALZHEMED (NC-531), PPI-368 (JP-T-11-514333), PPI-558 (JP-T-2001-500852), SKF-74652 *(*Biochem. J., 340(1), 283-289 (1999)), *etc,*), a cerebral function activator (e.g., aniracetam, nicergoline, *etc.),* a dopamine receptor agonist (e.g., L-dopa, bromocriptine, pergolide, talipexole, pramipexole, cabergoline, amantadine, *etc.*)*,* a monoamine oxidase (MAO) inhibitor (e.g., safrazine, deprenyl, selegiline, ramacemide, riluzole, *etc.),* an anticholinergic drug (e.g., trihexyphenidyl, biperiden, *etc.*)*,* a COMT inhibitor (e.g., entacapone, *etc.),* a therapeutic agent for amyotrophic lateral sclerosis (e.g., riluzole, a neurotrophic factor, *etc.),* a statin-based therapeutic agent for hyperlipidemia (e.g., sodium pravastatin, atorvastatin, simvastatin, rosuvastatin, *etc.),* a fibrate-based therapeutic agent for hyperlipidemia (e.g., clofibrate, *etc.*)*,* an apoptosis inhibitor (e.g., CPI-1189, IDN-6556, CEP-1347, *etc.),* a nerve differentiation and regeneration promoter (e.g., Leteprinim, Xaliproden (SR-57746-A), SB-216763, *etc.*)*,* a non-steroidal anti-inflammatory drug (e.g., meloxicam, tenoxicam, indomethacin, ibuprofen, celecoxib, rofecoxib, aspirin, indomethacin, *etc.),* a steroid drug (e.g., dexamethasone, hexestrol, cortisone acetate, *etc.),* a sexual hormone or derivatives thereof (e.g., progesterone, estradiol, estradiol benzoate, *etc.),* or the like. Furthermore, it may be also combined with a nicotinic receptor regulator, a γ-secretase inhibitor, a β-amyloid vaccine, a β-amyloid degrading enzyme, a squalene synthetase inhibiting agent, a therapeutic agent for the abnormal behavior, wandering or the like associated with progress of dementia, a hypotensor, a therapeutic agent for diabetes mellitus, an antidepressant, an antianxiety agent, a disease-modifying antirheumatoid agent, an anticytokine agent (e.g., a TNF inhibitor, a MAP kinase inhibitor, *etc.*), a parathyroid hormone (PTH), a calcium receptor antagonist or the like.

These combination medicaments are only exemplary and are not limited to these. The administration methods of these combination medicaments are not particularly limited, they may be oral administration or parenteral administration. Also, these medicaments may be administered in any combination of two or more. Furthermore, the medicaments for combined use include those that have been discovered as well as those that are to be discovered afterward, based on the mechanism described above.

Toxicity:

Toxicity of the capsule comprising (2R)-2-propyloctanoic acid or a salt thereof in the present invention is very low, and it is considered to be sufficiently safe for the use as a pharmaceutical drug.

Effect of the invention

A soft capsule comprising an oral irritative compound, particularly (2R)-2-propyloctanoic acid or a salt thereof which is useful as an agent for prevention, treatment and/or suppression of symptom progression for various neurodegenerative diseases and the like, can be provided by the present invention. Since such a soft capsule has a property of easily disintegrating in the stomach and not easily leaking the contents at the time of mastication, it is an excellent pharmaceutical preparation which can be internally taken safely without feeling acridness, pain, burning sensation and the like in the oral cavity and can show its pharmacological effects through its quick absorption, for example, even when

an old person or the like masticated it by mistake at the time of its taking.

In addition, since the soft capsule to be provided by the present invention can withstand its long-term preservation without causing dissolution delay, it can be offered to the clinical field as a soft capsule which maintains excellent quality.

## BEST MODE FOR CARRYING OUT THE INVENTION

The present invention is described below in detail based on Examples, but the present invention is not limited thereto. In addition, these may be changed within the range of not departing the scope of the present invention.

In this specification, in addition to the soft capsule which comprises (2R)-2-propyloctanoic acid, results of examinations using a soft capsule that comprises middle-chain fatty acid triglycerides (medium-chain triglycerides: MCT) are also disclosed. Although MCT is not an oral irritative compound, the fact that the soft capsule comprising MCT and the soft capsule comprising (2R)-2-propyloctanoic acid have the same property of easily disintegrating in the stomach and not easily leaking the contents at the time of mastication supports the effect of the present invention that the contents of the soft capsule may be any compounds.

### Example 1

Production of soft capsule containing (2R)-2-propyloctanoic acid (300 mg):

#### Example 1-1

Capsule shell composition Bovine gelatin:glycerine = 100:30

Bovine gelatin (20 kg) and concentrated glycerine (6 kg) were mixed at 70°C in the presence of purified water (20 kg) to obtain a uniform solution. This solution and (2R)-2-propyloctanoic acid (0.9 kg) were put into an encapsulator for soft capsules (a rotary type soft capsule making machine Model H-1; manufactured by Kamata) to obtain "capsules before drying" of (2R)-2-propyloctanoic acid-encapsulated soft capsules. By subjecting the thus obtained "capsules before drying" to a tumbler drying (24°C, 3 hours) and a tray drying (29°C, 15 to 45 hours) in this order, soft capsules (2100 capsules) containing 300 mg of (2R)-2-propyloctanoic acid in one capsule were obtained. By further carrying out the same operation 6 times, a total of 7 lots of the soft capsules were obtained. The tray drying time of respective lots was 27 hours in the case of lot # 1 to # 5, 15 hours in the case of lot # 6, and 45 hours in the case of lot # 7.

#### Example 1-2

Capsule shell composition Swine gelatin:glycerine = 100:30

Swine gelatin (20 kg) and concentrated glycerine (6 kg) were mixed at 75°C in the presence of purified water (16 kg) to obtain a uniform solution. This solution and (2R)-2-propyloctanoic acid (1.8 kg) were put into an encapsulator for soft capsules (a rotary type soft capsule making machine Model H-1; manufactured by Kamata) to obtain "capsules before drying" of (2R)-2-propyloctanoic acid-encapsulated soft capsules. By subjecting the thus obtained "capsules before drying" to a tumbler drying (23.5°C, 3 hours) and a tray drying (29°C, 27 hours) in this order, soft capsules (5700 capsules) containing 300 mg of (2R)-2-propyloctanoic acid in one capsule were obtained.

### Example 2

Production of soft capsule containing (2R)-2-propyloctanoic acid (100 mg):

#### Example 2-1

Capsule shell composition Bovine gelatin: glycerine = 100:30

Bovine gelatin (15 kg) and concentrated glycerine (4.5 kg) were mixed at 70°C in the presence of purified water (15 kg) to obtain a uniform solution. This solution and (2R)-2-propyloctanoic acid (1.0 kg) were put into an encapsulator for soft capsules (a rotary type soft capsule making machine Model H-1; manufactured by Kamata) to obtain "capsules before drying" of (2R)-2-propyloctanoic acid-encapsulated soft capsules. By subjecting the thus obtained "capsules before drying" to a tumbler drying (22°C, 4 hours) and a tray drying (28.5°C, 26 hours) in this order, soft capsules (3000 capsules) containing 100 mg of (2R)-2-propyloctanoic acid in one capsule were obtained. By further carrying out the same operation once, a total of 2 lots of the soft capsules were obtained.

Example 2-2

Capsule shell composition Swine gelatin:glycerine = 100:30

Swine gelatin (20 kg) and concentrated glycerine (6 kg) were mixed at 75°C in the presence of purified water (16 kg) to obtain a uniform solution. This solution and (2R)-2-propyloctanoic acid (1.2 kg) were put into an encapsulator for soft capsules (a rotary type soft capsule making machine Model H-1; manufactured by Kamata) to obtain "capsules before drying" of (2R)-2-propyloctanoic acid-encapsulated soft capsules. By subjecting the thus obtained "capsules before drying" to a tumbler drying (23.5°C, 3 hours) and a tray drying (29°C, 27 hours) in this order, soft capsules (5300 capsules) containing 100 mg of (2R)-2-propyloctanoic acid in one capsule were obtained.

Example 3

Measurement of capsule shell thickness of (2R)-2-propyloctanoic acid-containing soft capsule:

The capsule shell thickness (shell thickness, first seam thickness, second seam thickness) was measured in accordance with the following method, on the (2R)-2-propyloctanoic acid-containing soft capsules produced in Example 1 and Example 2 and MCT-containing soft capsules produced in the same manner using middle-chain fatty acid triglycerides (medium-chain triglycerides: MCT) instead of (2R)-2-propyloctanoic acid.

<Method>

(1) Central part of the capsule is cut in round slices using a knife.
(2) The inclusion liquid is washed out using n-hexane and quickly wiped off using a piece of cloth.
(3) The thickness of capsule shell is measured by observing the cut plane under an optical microscope, using a scale as the standard.
(4) By measuring thickness of each part (shell thickness, first seam thickness, second seam thickness) on 5 to 10 samples for each lot, the maximum value (Max), minimum value (Min) and average value (Mean) are calculated.

<Results>

Results of the (2R)-2-propyloctanoic acid (300 mg)-containing soft capsule produced using bovine gelatin are shown in Table 1, and results of the MCT-containing soft capsule produced in the same manner in Table 2, results of the (2R)-2-propyloctanoic acid (100 mg)-containing soft capsule produced using bovine gelatin and of the MCT-containing soft capsule produced in the same manner in Table 3, and results of the soft capsule produced using swine gelatin in Table 4. In this specification, the expression of "Active" in the following tables means the soft capsule which contains (2R)-2-propyloctanoic acid as the inclusion liquid, and the expression of "MCT" means the soft capsule which contains MCT as the inclusion liquid.

Table 1

| | Lot | Active (300 mg) | | | | | | | |
|---|---|---|---|---|---|---|---|---|---|
| | | #1 | #2 | #3 | #4 | #5 | #6 | #7 | total |
| Shell thickness (mm) | Mean | 0.256 | 0.287 | 0.175 | 0.176 | 0.168 | 0.172 | 0.171 | 0.201 |
| | Max | 0.278 | 0.320 | 0.188 | 0.190 | 0.180 | 0.178 | 0.180 | 0.320 |
| | Min | 0.220 | 0.260 | 0.160 | 0.145 | 0.155 | 0.165 | 0.162 | 0.145 |
| First seam thickness (mm) | Mean | 0.314 | 0.366 | 0.218 | 0.188 | 0.262 | 0.206 | 0.233 | 0.255 |
| | Max | 0.362 | 0.411 | 0.249 | 0.194 | 0.296 | 0.214 | 0.247 | 0.411 |
| | Min | 0.280 | 0.335 | 0.194 | 0.183 | 0.224 | 0.189 | 0.222 | 0.183 |
| Second seam thickness (mm) | Mean | 0.219 | 0.266 | 0.167 | 0.163 | 0.148 | 0.156 | 0.162 | 0.183 |
| | Max | 0.248 | 0.300 | 0.187 | 0.171 | 0.154 | 0.165 | 0.169 | 0.300 |
| | Min | 0.169 | 0.239 | 0.142 | 0.152 | 0.140 | 0.150 | 0.152 | 0.140 |

Table 2

| | | MCT (300 mg) | | | | |
|---|---|---|---|---|---|---|
| | Lot | # 1 | # 2 | #3 | # 4 | total |
| Shell thickness (mm) | Mean | 0.281 | 0.296 | 0.186 | 0.184 | 0.237 |
| | Max | 0.333 | 0.308 | 0.197 | 0.198 | 0.333 |
| | Min | 0.240 | 0.278 | 0.177 | 0.160 | 0.160 |
| First seam thickness (mm) | Mean | 0.321 | 0.405 | 0.265 | 0.257 | 0.312 |
| | Max | 0.356 | 0.452 | 0.334 | 0.311 | 0.452 |
| | Min | 0.309 | 0.379 | 0.224 | 0.229 | 0.224 |
| Second seam thickness (mm) | Mean | 0.253 | 0.260 | 0.174 | 0.168 | 0.214 |
| | Max | 0.268 | 0.303 | 0.187 | 0.179 | 0.303 |
| | Min | 0.233 | 0.242 | 0.156 | 0.159 | 0.156 |

The (2R)-2-propyloctanoic acid (300 mg)-containing soft capsule produced using bovine gelatin was from 0.145 to 0320 mm in shell thickness, from 0.183 to 0.411 mm in first seam thickness, and from 0.140 to 0.300 mm in second seam thickness. Also, the MCT-containing soft capsule produced in the same manner was from 0.160 to 0.333 mm in shell thickness, from 0.224 to 0.452 mm in first seam thickness, and from 0.155 to 0.303 mm in second seam thickness.

Table 3

| | | Active (100 mg) | | | MCT (100 mg) | | |
|---|---|---|---|---|---|---|---|
| | Lot | #1 | #2 | total | #1 | # 2 | total |
| Shell thickness (mm) | Mean | 0.264 | 0.337 | 0.300 | 0.260 | 0.355 | 0.308 |
| | Max | 0.300 | 0.365 | 0.365 | 0.283 | 0.388 | 0.388 |
| | Min | 0.230 | 0.322 | 0.230 | 0.248 | 0.322 | 0.248 |
| First seam thickness (mm) | Mean | 0.206 | 0.327 | 0.266 | 0.231 | 0.362 | 0.296 |
| | Max | 0.213 | 0.362 | 0.362 | 0.251 | 0.400 | 0.400 |
| | Min | 0.201 | 0.292 | 0.201 | 0.204 | 0.338 | 0.204 |
| Second seam thickness (mm) | Mean | 0.239 | 0.293 | 0.266 | 0.268 | 0.272 | 0.270 |
| | Max | 0.274 | 0.353 | 0.353 | 0.300 | 0.306 | 0.306 |
| | Min | 0.210 | 0.251 | 0.210 | 0.248 | 0.230 | 0.230 |

The (2R)-2-propyloctanoic acid (100 mg)-containing soft capsule produced using bovine gelatin was from 0.230 to 0.365 mm in shell thickness, from 0.201 to 0.362 mm in first seam thickness, and from 0.210 to 0.353 mm in second seam thickness. Also, the MCT-containing soft capsule produced in the same manner was from 0.248 to 0.388 mm in shell thickness, from 0.204 to 0.400 mm in first seam thickness, and from 0.230 to 0.306 mm in second seam thickness.

Table 4

| | | Active (300 mg) | MCT (300 mg) | Active (100 mg) | MCT (100 mg) |
|---|---|---|---|---|---|
| Shell thickness (mm) | Mean | 0.179 | 0.203 | 0.227 | 0.256 |
| | Max | 0.205 | 0.212 | 0.243 | 0.275 |
| | Min | 0.157 | 0.193 | 0.212 | 0.213 |

(continued)

| | | Active (300 mg) | MCT (300 mg) | Active (100 mg) | MCT (100 mg) |
|---|---|---|---|---|---|
| First seam thickness (mm) | Mean | 0.263 | 0.289 | 0.218 | 0.223 |
| | Max | 0.294 | 0.329 | 0.235 | 0.243 |
| | Min | 0.222 | 0.268 | 0.208 | 0.187 |
| Second seam thickness (mm) | Mean | 4.165 | 0.184 | 0.248 | 0.264 |
| | Max | 0.179 | 0.198 | 0.257 | 0.298 |
| | Min | 0.154 | 0.173 | 0.237 | 0.241 |

The (2R)-2-propyloctanoic acid (300 mg)-containing soft capsule produced using swine gelatin was from 0.157 to 0.205 mm in shell thickness, from 0.222 to 0.294 mm in first seam thickness, and from 0.154 to 0.179 mm in second seam thickness. Also, the MCT-containing soft capsule produced in the same manner was from 0.193 to 0.212 mm in shell thickness, from 0.268 to 0.329 mm in first seam thickness, and from 0.173 to 0.198 mm in second seam thickness.

The (2R)-2-propyloctanoic acid (100 mg)-containing soft capsule produced using swine gelatin was from 0.212 to 0.243 mm in shell thickness, from 0.208 to 0.235 mm in first seam thickness, and from 0.237 to 0.257 mm in second seam thickness. Also, the MCT-containing soft capsule produced in the same manner was from 0.213 to 0.275 mm in shell thickness, from 0.187 to 0.243 mm in first seam thickness, and from 0.241 to 0.298 mm in second seam thickness.

Example 4

Measurement of capsule shell water content of (2R)-2-propyloctanoic acid-containing soft capsule:

The capsule shell water content was measured in accordance with the following method, on the (2R)-2-propyloctanoic acid-containing soft capsules produced in Example 1 and Example 2 and MCT-containing soft capsules produced in the same manner using MCT instead of (2R)-2-propyloctanoic acid.

<Method>

(1) A 10 ml capacity weighing bottle is prepared and heated at 105°C in advance.
(2) The capsule is cut open using scissors in a solvent (n-hexane) to washout the inclusion liquid.
(3) The solvent is wiped off with a piece of cloth, and the capsule shell is cut into pieces of about 2 to 4 mm using scissors.
(4) The same operation is carried out on 10 to 20 samples of each lot.
(5) About 1 g of the finely cut capsule shell is put into the weighing bottle to measure the sample weight before drying, and then dried at 105°C for 2 hours.
(6) The sample weight after drying is measured, and the water content is calculated using the following formula.

$$\text{Water content (\%)} = ((\text{weight before drying} - \text{weight after drying})/\text{weight before drying}) \times 100$$

<Results>

Results of the (2R)-2-propyloctanoic acid (300 mg)-containing soft capsule produced using bovine gelatin are shown in Table 5, and results of the MCT-containing soft capsule produced in the same manner in Table 6, results of the (2R)-2-propyloctanoic acid (100 mg)-containing soft capsule produced using bovine gelatin and of the MCT-containing soft capsule produced in the same manner in Table 7, and results of the soft capsule produced using swine gelatin in Table 8. In this specification, similar to the above-described case, the expression of "Active" means the soft capsule which contains (2R)-2-propyloctanoic acid as the inclusion liquid, and the expression of "MCT" means the soft capsule which contains MCT as the inclusion liquid.

Table 5

| | | Active (300 mg) | | | | | | | |
|---|---|---|---|---|---|---|---|---|---|
| | Lot | #1 | #2 | #3 | #4 | #5 | #6 | #7 | Mean |
| Water content | (%) | 5.6 | 6.0 | 7.3 | 7.2 | 7.2 | 6.4 | 6.8 | 6.6 |

Table 6

| | | MCT (300 mg) | | | | |
|---|---|---|---|---|---|---|
| | Lot | # 1 | # 2 | # 3 | # 4 | Mean |
| Water content | (%) | 5.8 | 6.4 | 6.5 | 6.4 | 6.3 |

Water content of the (2R)-2-propyloctanoic acid (300 mg)-containing soft capsule produced using bovine gelatin was from 5.6 to 7.3% (6.6% in average among lots). Also, water content of the soft capsule produced in the same manner was from 5.8 to 6.5% (6,3% in average among lots).

Table 7

| | | Active (100 mg) | | | MCT (100 mg) | | |
|---|---|---|---|---|---|---|---|
| | Lot | #1 | #2 | Mean | #1 | #2 | Mean |
| Water content | (%) | 5.6 | 6.9 | 6.3 | 5.9 | 7.0 | 6.5 |

Water content of the (2R)-2-propyloctanoic acid (100 mg)-containing soft capsule produced using bovine gelatin was from 5.6 to 6.9% (6.3% in average among lots). Also, water content of the soft capsule produced in the same manner was from 5.9 to 7.0% (6.5% in average among lots).

Table 8

| | Active (300 mg) | MCT (300 mg) | Active (100 mg) | MCT (100 mg) |
|---|---|---|---|---|
| Water content (%) | 6.9 | 7.1 | 6.7 | 6.8 |

Water content of the (2R)-2-propyloctanoic acid (300 mg)-containing soft capsule produced using swine gelatin was 6.9%. Also, water content of the soft capsule produced in the same manner was 7.1%.

Water content of the (2R)-2-propyloctanoic acid (100 mg)-containing soft capsule produced using swine gelatin was 6.7%. Also, water content of the soft capsule produced in the same manner was 6.8%.

Example 5

Measurement of the strength of (2R)-2-propyloctanoic acid-containing soft capsule:

The capsule strength (cracking load) was measured in accordance with the following method, on the (2R)-2-propyloctanoic acid-containing soft capsules produced in Example 1 and Example 2 and MCT-containing soft capsules produced in the same manner using MCT instead of (2R)-2-propyloctanoic acid. <Method>

(1) Each capsule is allowed to stand still on the measuring part of a small bench type test machine manufactured by Shimadzu Corp. (EZ Test-500N), in such a manner that the ground plane and the adhering plane of the capsule come parallel.
(2) Pressurization is carried out at a rate of 20 mm/min using a compression hardware of 20 mm in diameter.
(3) The load when the capsule is broken (maximum point load) is measured on 5 samples for each lot, and the average value (Mean) is calculated.

<Results>

Results of the (2R)-2-propyloctanoic acid (300 mg)-containing soft capsule produced using bovine gelatin are shown in Table 9, and results of the MCT-containing soft capsule produced in the same manner in Table 10, results of the (2R)-2-propyloctanoic acid (100 mg)-containing soft capsule produced using bovine gelatin and of the MCT-containing soft capsule produced in the same manner in Table 11, and results of the soft capsule produced using swine gelatin in Table 12. In this specification, similar to the above-described case, the expression of "Active" means the soft capsule which contains (2R)-2-propyloctanoic acid as the inclusion liquid, and the expression of "MCT" means the soft capsule which contains MCT as the inclusion liquid.

Table 9

| | | Active (300 mg) | | | | | | | |
|---|---|---|---|---|---|---|---|---|---|
| | Lot | # 1 | #2 | #3 | #4 | #5 | #6 | #7 | Mean |
| Strength | (N) | 288.8 | 289.1 | 297.9 | 293.7 | 280.6 | 306.2 | 303.3 | 294.2 |

Table 10

| | | MCT (300 mg) | | | | |
|---|---|---|---|---|---|---|
| | Lot | # 1 | # 2 | # 3 | # 4 | Mean |
| Strength | (N) | 283.0 | 331.3 | 268.5 | 278.0 | 290.2 |

Strength of the (2R)-2-propyloctanoic acid (300 mg)-containing soft capsule produced using bovine gelatin was from 280.6 to 306.2 N (294.2 N in average among lots). Also, strength of the MCT-containing soft capsule produced in the same manner was from 268.5 to 331.3 N (290.2 N in average among lots).

Table 11

| | | Active (100 mg) | | | MCT (100 mg) | | |
|---|---|---|---|---|---|---|---|
| | Lot | #1 | #2 | Mean | #1 | #2 | Mean |
| Strength | (N) | 193.5 | 255.8 | 224.7 | 196.7 | 209.5 | 203.1 |

Strength of the (2R)-2-propyloctanoic acid (100 mg)-containing soft capsule produced using bovine gelatin was from 193.5 to 255.8 N (224.7 N in average among lots). Also, strength of the MCT-containing soft capsule produced in the same manner was from 196.7 to 209.5 N (203.1 N in average among lots).

Table 12

| | | Active (300 mg) | MCT (300 mg) | Active (100 mg) | MCT (100 mg) |
|---|---|---|---|---|---|
| Strength | (N) | 273.1 | 289.2 | 235.1 | 274.7 |

Strength of the (2R)-2-propyloctanoic acid (300 mg)-containing soft capsule produced using swine gelatin was 273.1 N. Also, strength of the soft capsule produced in the same manner was 289.2 N.
Strength of the (2R)-2-propyloctanoic acid (100 mg)-containing soft capsule produced using swine gelatin was 235.1 N. Also, strength of the soft capsule produced in the same manner was 274.7 N.

Example 6

Measurement of the disintegration time of (2R)-2-propyloctanoic acid-containing soft capsule:

The capsule disintegration time of 6 samples of each lot was measured in accordance with the disintegration test (capsules) described in the 14th edition of Japanese Pharmacopoeia, on the (2R)-2-propyloctanoic acid-containing soft capsules produced in Example 1 and Example 2 and MCT-containing soft capsules produced in the same manner using MCT instead of (2R)-2-propyloctanoic acid, and the average value (Mean) was calculated.

<Results>

Results of the (2R)-2-propyloctanoic acid (300 mg)-containing soft capsule produced using bovine gelatin are shown in Table 13, and results of the MCT-containing soft capsule produced in the same manner in Table 14, results of the (2R)-2-propyloctanoic acid (100 mg)-containing soft capsule produced using bovine gelatin and of the MCT-containing soft capsule produced in the same manner in Table 15, and results of the soft capsule produced using swine gelatin in Table 16. In this specification, similar to the above-described case, the expression of "Active" means the soft capsule which contains (2R)-2-propyloctanoic acid as the inclusion liquid, and the expression of "MCT" means the soft capsule which contains MCT as the inclusion liquid. Table 13

| | | Active (300 mg) | | | | | | | |
|---|---|---|---|---|---|---|---|---|---|
| | Lot | #1 | #2 | #3 | #4 | #5 | #6 | #7 | Mean |
| Disintegration time | (min) | 6.0 | 5.8 | 6.3 | 5.7 | 6.3 | 6.0 | 6.2 | 6.0 |

Table 14

| | | MCT (300 mg) | | | | |
|---|---|---|---|---|---|---|
| | Lot | #1 | #2 | #3 | #4 | Mean |
| Disintegration time | (min) | 6.2 | 6.5 | 7.5 | 7.3 | 6.9 |

Disintegration time of the (2R)-2-propyloctanoic acid (300 mg)-containing soft capsule produced using bovine gelatin was from 5.7 to 6.3 minutes (6.0 minutes in average among lots). Also, disintegration time of the MCT-containing soft capsule produced in the same manner was from 6.2 to 7.5 minutes (6.9 minutes in average among lots). Table 15

| | | Active (100 mg) | | | MCT (100 mg) | | |
|---|---|---|---|---|---|---|---|
| | Lot | # 1 | # 2 | Mean | # 1 | #2 | Mean |
| Disintegration time | (min) | 5.8 | 6.0 | 5.9 | 6.0 | 7.0 | 6.5 |

Disintegration time of the (2R)-2-propyloctanoic acid (100 mg)-containing soft capsule produced using bovine gelatin was from 5.8 to 6.0 minutes (5.9 minutes in average among lots). Also, disintegration time of the MCT-containing soft capsule produced in the same manner was from 6.0 to 7.0 minutes (6.5 minutes in average among lots).

Table 16

| | | Active (300 mg) | MCT (300 mg) | Active (100 mg) | MCT (100 mg) |
|---|---|---|---|---|---|
| Disintegration time | (min) | 5.5 | 6.8 | 6.2 | 7.2 |

Disintegration time of the (2R)-2-propyloctanoic acid (300 mg)-containing soft capsule produced using swine gelatin was 5.5 minutes. Also, disintegration time of the MCT-containing soft capsule produced in the same manner was 6.8 minutes.

Disintegration time of the (2R)-2-propyloctanoic acid (100 mg)-containing soft capsule produced using swine gelatin was 6.2 minutes. Also, disintegration time of the MCT-containing soft capsule produced in the same manner was 7.2 minutes.

Example 7

Mastication test of (2R)-2-propyloctanoic acid-containing soft capsule:

Difficulty in crushing the capsule with the teeth was evaluated in accordance with the following method, on the (2R)-2-propyloctanoic acid-containing soft capsules produced in Example 1 and Example 2 and MCT-containing soft capsules produced in the same manner using MCT instead of (2R)-2-propyloctanoic acid.

<Method>

(1) Soft capsules are crushed with the teeth by 10 monitors without swallowing.
(2) Difficulty in crushing them with the teeth is evaluated by three steps (can be crushed with the teeth easily or markedly easily; can hardly be crushed with the teeth; cannot be crushed with the teeth).

<Results>

Results of the soft capsules produced using bovine gelatin are shown in Table 17, and results of the soft capsules produced using swine gelatin in Table 18. In this specification, similar to the above-described case, the expression of "Active" means the soft capsule which contains (2R)-2-propyloctanoic acid as the inclusion liquid, and the expression of "MCT" means the soft capsule which contains MCT as the inclusion liquid.

Table 17

| | Bovine gelatin | | | |
|---|---|---|---|---|
| | Active (300 mg) | MCT (300 mg) | Active (100 mg) | MCT (100 mg) |
| Cannot be crushed | 0 | 0 | 1 | 1 |
| Can hardly be crushed | 10 | 10 | 9 | 9 |
| Can be crushed easily or markedly easily | 0 | 0 | 0 | 0 |

Table 18

| | Swine gelatin | | | |
|---|---|---|---|---|
| | Active (300 mg) | MCT (300 mg) | Active (100 mg) | MCT (100 mg) |
| Cannot be crushed | 0 | 1 | 0 | 1 |
| Can hardly be crushed | 10 | 9 | 10 | 9 |
| Can be crushed easily or markedly easily | 0 | 0 | 0 | 0 |

Both of the (2R)-2-propyloctanoic acid-containing soft capsules produced in Example 1 and Example 2 and MCT-containing soft capsules produced in the same manner using MCT instead of (2R)-2-propyloctanoic acid can be hardly crushed with the teeth or cannot be crushed with the teeth. Accordingly, it was judged that these soft capsules are stable to mastication.

Example 8

Dissolution delay evaluation test of (2R)-2-propyloctanoic acid-containing soft capsule:

Soft capsules containing (2R)-2-propyloctanoic acid were produced in accordance with the following formulations 1A to 5A (300 mg preparations) and formulations 1B to 4B (100 mg preparations), and the presence or absence of dissolution delay was verified by carrying out dissolution tests (1) just after their production, (2) after a severe test and (3) after a long-term stability test.

(Formulation 1A) Bovine gelatin:glycerine:sorbitol = 100:10:30

Bovine gelatin (25 kg), concentrated glycerine (2.5 kg) and 70% D-sorbitol solution (7.5 kg) were mixed at 70°C in the presence of purified water (25 kg), thereby obtaining a uniform solution. This solution and (2R)- 2-propyloctanoic acid (0.7 kg) were put into an encapsulator for soft capsules (a rotary type soft capsule making machine Model H-1; manufactured by Kamata) to obtain "capsules before drying" of (2R)-2-propyloctanoic acid-encapsulated soft capsules. By subjecting the thus obtained "capsules before drying" to a tumbler drying (23°C, 3 hours) and a tray drying (30°C, 27 hours) in this order, the title soft capsules (2800 capsules) containing 300 mg of (2R)-2-propyloctanoic acid in one

capsule were obtained.

(Formulation 2A) Bovine gelatin: glycerine = 100:20

Bovine gelatin (25 kg) and concentrated glycerine (5 kg) were mixed at 70°C in the presence of purified water (25 kg), thereby obtaining a uniform solution. This solution and (2R)-2-propyloctanoic acid (0.9 kg) were put into the encapsulator for soft capsules (a rotary type soft capsule making machine Model H-1; manufactured by Kamata) to obtain "capsules before drying" of (2R)-2-propyloctanoic acid-encapsulated soft capsules. By subjecting the thus obtained "capsules before drying" to a tumbler drying (22.5°C, 3 hours) and a tray drying (3I°C, 27 hours) in this order, the title soft capsules (2200 capsules) containing 300 mg of (2R)-2-propyloctanoic acid in one capsule were obtained.

(Formulation 3A) Bovine gelatin:glycerine = 100:25

By using bovine gelatin (20 kg), concentrated glycerine (5 kg), purified water (20 kg) and (2R)-2-propyloctanoic acid (1.2 kg), and subjecting them to the same operation of formulation 2A, the title soft capsules (2200 capsules) were obtained.

(Formulation 4A: the same shell composition of Example 1-1) Bovine gelatin:glycerine = 100:30

By using bovine gelatin (20 kg), concentrated glycerine (6 kg), purified water (20 kg) and (2R)-2-propyloctanoic acid (0.9 kg), and subjecting them to the same operation of formulation 2A, the title soft capsules (2200 capsules) were obtained.

(Formulation 5A) Bovine gelatin:glycerine = 100:40

By using bovine gelatin (20 kg), concentrated glycerine (8 kg), purified water (20 kg) and (2R)-2-propyloctanoic acid (0.9 kg), and subjecting them to the same operation of formulation 2A, the title soft capsules (2100 capsules) were obtained.

(Formulation 1B) Bovine gelatin:glycerine:sorbitol = 100:20:20

Bovine gelatin (25 kg), concentrated glycerine (5 kg) and 70% D-sorbitol solution (5 kg) were mixed at 70°C in the presence of purified water (25 kg), thereby obtaining a uniform solution. This solution and (2R)- 2-propyloctanoic acid (6.4 kg) were put into the encapsulator for soft capsules (a rotary type soft capsule making machine Model H-1; manufactured by Kamata) to obtain "capsules before drying" of (2R)-2-propyloctanoic acid-encapsulated soft capsules. By subjecting the thus obtained "capsules before drying" to a tumbler drying (24.5°C, 6 hours) and a tray drying (30°C, 18 hours) in this order, the title soft capsules (58800 capsules) containing 100 mg of (2R)-2-propyloctanoic acid in one capsule were obtained.

(Formulation 2B) Bovine gelatin:glycerine = 100:25

Bovine gelatin (20 kg) and concentrated glycerine (5 kg) were mixed at 70°C in the presence of purified water (20 kg), thereby obtaining a uniform solution. This solution and (2R)-2-propyloctanoic acid (1.2 kg) were put into the encapsulator for soft capsules (a rotary type soft capsule making machine Model H-1; manufactured by Kamata) to obtain "capsules before drying" of (2R)-2-propyloctanoic acid-encapsulated soft capsules. By subjecting the thus obtained "capsules before drying" to a tumbler drying (23.5°C, 4 hours) and a tray drying (28.5°C, 26 hours) in this order, the title soft capsules (3000 capsules) containing 100 mg of (2R)-2-propyloctanoic acid in one capsule were obtained.

(Formulation 3B: the same shell composition of Example 2-1) Bovine gelatin: glycerine = 100:30

By using bovine gelatin (15 kg), concentrated glycerine (4.5 kg), purified water (15 kg) and (2R)-2-propyloctanoic acid (0.6 kg), and subjecting them to the same operation of formulation 2B, the title soft capsules (3000 capsules) were obtained.

(Formulation 4B) Bovine gelatin:glycerine = 100:40

By using bovine gelatin (15 kg), concentrated glycerine (6 kg), purified water (15 kg) and (2R)-2-propyloctanoic acid (0.6 kg), and subjecting them to the same operation of formulation 2B, the title soft capsules (4500 capsules) were

obtained.

(1) Dissolution test just after the production

Dissolution test was carried out quickly after production of the soft capsules produced based on the formulations 1 A to 5A (300 mg preparations) and formulations 1B to 4B (100 mg preparations), under the following test conditions.

<Dissolution test>

(Test method)

The dissolution test second method (paddle method), the 14th edition of Japanese Pharmacopoeia

(Test conditions)

Number of revolution: 50 rpm (100 mg preparations), 75 rpm (300 mg preparations); Test liquid: 0.05 mol/l disodium hydrogenphosphate/0.025 mol/l citric acid buffer liquid (pH 8.0);
Liquid volume: 900 ml; and
Temperature: 37°C.

<Results>

Results of the dissolution test (dissolution ratio (%)) carried out quickly after production of the soft capsules produced based on the formulations 1A to 5A (300 mg preparations) and formulations 1B to 4B (100 mg preparations) are shown in the following Table 19 and Table 20.

Table 19

| Time (min) | Formulation 1A | Formulation 2A | Formulation 3A | Formulation 4A | Formulation 5A |
|---|---|---|---|---|---|
| 0 | 0.0 | 0.0 | 0.0 | 0.0 | 0.0 |
| 30 | 100.0 | 99.6 | 99.6 | 99.5 | 99.4 |

Table 20

| Time (min) | Formulation 1B | Formulation 2B | Formulation 3B | Formulation 4B |
|---|---|---|---|---|
| 0 | 0.0 | 0.0 | 0.0 | 0.0 |
| 30 | 95.9 | 99.4 | 98.1 | 98.7 |

The formulations 1A, 2A, 3A, 4A and 5A showed dissolution ratios of 100.0%, 99.6%, 99.6%, 99.5% and 99.4%, respectively, 30 minutes after commencement of the dissolution test. Also, the formulations 1B, 2B, 3B and 4B showed dissolution ratios of 95.9%, 99.4%, 98.1% and 98.7%, respectively, 30 minutes after commencement of the test.
Each of the soft capsules produced based on the formulations 1A to 5A (300 mg preparations) and formulations 1B to 4B (100 mg preparations) was a quick dissolution soft capsule.

(2) Dissolution test after a severe testing (50°C, 2 weeks or 1 month)

Each of the soft capsules produced based on the formulations 2A to 5A (300 mg preparations) and formulations 2B to 4B (100 mg preparations) was subjected to a severe testing (50°C, 1 month for the 300 mg preparations; 50°C, 2 weeks for the 100 mg preparations), and then the dissolution test described in (1) was carried out.

<Results>

Results of the dissolution test (dissolution ratio (%)) carried out after the severe test of the soft capsules produced based on the formulations 2A to 5A (300 mg preparations) and formulations 2B to 4B (100 mg preparations) are shown in Table 21 and Table 22. In this case, as a measure of the initial values, initial value of the formulation 3A is shown in

the table for the 300 mg preparations, and initial value of the formulation 3B for the 100 mg preparations. In this specification, the numerical value described in the lower side parentheses for the dissolution ratio after 30 minutes shows the ratio of change (%) based on the initial value of each formulation.

Table 21

| Time (min) | Initial value (Formulation 3A) | Formulation 2A | Formulation 3A | Formulation 4A | Formulation 5A |
|---|---|---|---|---|---|
| 0 | 0.0 | crack | 0.0 | 0.0 | 0.0 |
| 30 | 99.6 | crack | 92.8 (6.8) | 88.0 (11.6) | 34.4 (65.4) |

Table 22

| Time (min) | Initial value (Formulation 3B) | Formulation 2B | Formulation 3B | Formulation 4B |
|---|---|---|---|---|
| 0 | 0.0 | 0.0 | 0.0 | 0.0 |
| 30 | 98.1 | 98.2 (1.2) | 90.7 (7.5) | 91.4 (7.4) |

Regarding the formulation 2A, its dissolution test was not carried out because of the generation of cracking during the severe test. The formulations 3A, 4A and 5A showed dissolution ratios of 92.8%, 88.0% and 34.4%, respectively, 30 minutes after commencement of the dissolution test. Also, the formulations 2B, 3B and 4B showed dissolution ratios of 98.2%, 90.7% and 91.4%, respectively, 30 minutes after commencement of the test.

The formulation 2A was unable to withstand the severe test, and dissolution delay was found in the case of the formulation 5A.

(3) Dissolution test after a long-term stability test (25°C or 30°C, about 1.5 years)

Each of the soft capsules produced based on the formulations 1A and 4A (300 mg preparations) and formulations 1B and 3B (100 mg preparations) was subjected to a long-term stability test (formulation 1A: 30°C, 18 months; formulation 4A: 30°C, 17 months; formulation 1B: 25°C, 18 months; formulation 3B: 25°C, 17 months), and then the dissolution test described in (1) was carried out.

<Results>

Results of the dissolution test (dissolution ratio (%)) carried out after the long-term stability test of the soft capsules produced based on the formulations 1A and 4A (300 mg preparations) and formulations 1B and 3B (100 mg preparations) are shown in the following Table 23 and Table 24. In this specification, the numerical value described in the lower side parentheses for the dissolution ratio after 30 minutes shows the ratio of change (%) based on the initial value of each formulation.

Table 23

| Time (min) | Initial value (Formulation 1A) | 30°C, 18 months (Formulation 1A) | Initial value (Formulation 4A) | 30°C, 17 months (Formulation 4A) |
|---|---|---|---|---|
| 0 | 0.0 | 0.0 | 0.0 | 0.0 |
| 30 | 100.0 | 71.5 (28.5) | 99.5 | 95.3 (4.2) |

Table 24

| Time (min) | Initial value (Formulation 1B) | 30°C, 18 months (Formulation 1B) | Initial value (Formulation 3B) | 30°C, 17 months (Formulation 3B) |
|---|---|---|---|---|
| 0 | 0.0 | 0.0 | 0.0 | 0.0 |
| 30 | 95.9 | 93.3 (2-7) | 98.1 | 95.4 (2.8) |

The formulations 1A and 4A showed dissolution ratios of 71.5% and 95.3%, respectively, 30 minutes after commencement of the dissolution test. Also, the formulations 1B and 3B showed dissolution ratios of 93.3% and 95.4%, respectively, 30 minutes after commencement of the test.

Dissolution delay was found in the case of the formulation 1A.

From the results of the above dissolution tests, it was found that the formulation 1A (bovine gelatin:glycerine: sorbitol = 100:10:30) causes dissolution delay by the long-term stability test, the formulation 2A (bovine gelatin:glycerine = 100:20) causes cracking by the severe test, and the formulation 5A (bovine gelatin:glycerine = 100:40) causes dissolution delay by the severe test.

Also, in each case of the 300 mg preparations and 100 mg preparations, a shell formulation having a ratio of gelatin:glycerine = 100:30 was the formulation which most hardly causes dissolution delay by the dissolution test of (1) just after the production, (2) after the severe test and (3) after the long-term stability test.

That is, it was found that when the dissolution delay is used as the index in the case of a formulation of bovine gelatin and glycerine as a formulation which does not contain sorbitol, their ratio is preferably 25 parts by mass or more and 40 parts by mass or less (preferably less than 40 parts by mass) of glycerine relative to 100 parts by mass of gelatin, particularly preferably 30 parts by mass of glycerine relative to 100 parts by mass of gelatin.

In addition, since dissolution delay occurs by the long-term stability test when 30 parts by mass of sorbitol is used relative to 100 parts by mass of gelatin in a formulation of bovine gelatin, glycerine and sorbitol as a formulation which contains sorbitol, it was found that a pharmaceutical preparation which excels in preservation stability and hardly generates dissolution delay can be produced when the ratio of sorbitol is set to less than 30 parts by mass, preferably 20 parts by mass or less, relative to 100 parts by mass of gelatin.

INDUSTRIAL APPLICABILITY

As shown in the following, it is possible to apply the present invention to medicines.

Since the capsule of the present invention contains (2R)-2-propyloctanoic acid or a salt thereof, it can be used as an agent for prevention, treatment and/or suppression of symptom progression for various diseases of mammals such as neurodegenerative diseases (e.g., human, non-human animals such as monkey, sheep, bovine, equine, canine, feline, rabbit, rat, mouse, *etc.*).

Also, since the capsule of the present invention has a property of easily disintegrating in the stomach and not easily leaking the contents at the time of mastication, it can be internally taken safely without feeling acridness, pain, burning sensation and the like in the oral cavity and can show its pharmacological effects through its quick absorption, for example, even when an old person or the like masticated it by mistake at the time of its taking.

In addition, since the capsule of the present invention is a quickly dissolving soft capsule having such an excellent storage stability that it can withstand its long-term preservation without causing dissolution delay, it can show stable absorption property and exert stable pharmacological effects even under a long-term preservation.

## Claims

1. A capsule stable against mastication comprising (2R)-2-propyloctanoic acid or a salt thereof and having a property of following (1) and/or (2):

   (1) having a capsule shell comprising (a) at least one capsule base selected from a protein, a saccharide, a biodegradable plastic and a hydrogenated fat and (b) a plasticizer, and having a water content of 4.0 to 10.0%;
   (2) being easily disintegrated in the stomach.

2. The capsule stable against mastication according to claim 1, which is a soft capsule.

3. The soft capsule stable against mastication according to claim 2, which has at least one property selected from the following (A) to (F):

   (A) wherein it has a strength of 150 to 400 N by a cracking test;
   (B) wherein it has a disintegration time of 3 to 10 minutes by the disintegration test stipulated in Japanese Pharmacopoeia;
   (C) wherein the capsule shell has a shell thickness of 0.05 to 0.50 mm;
   (D) wherein the capsule shell has a first seam thickness of 0.10 to 0.55 mm;
   (E) wherein the capsule shell has a second seam thickness of 0.05 to 0.50 mm;
   (F) wherein the capsule shell has a water content of 5.0 to 9.0%.

4. The soft capsule stable against mastication according to claim 3, wherein the property is at least one selected from the following (A) to (F):

(A) wherein it has a strength of 180 to 350 N by a cracking test;
(B) wherein it has a disintegration time of 5 to 8 minutes by the disintegration test stipulated in Japanese Pharmacopoeia;
(C) wherein the capsule shell has a shell thickness of 0.10 to 0.45 mm;
(D) wherein the capsule shell has a first seam thickness of 0.15 to 0.50 mm;
(E) wherein the capsule shell has a second seam thickness of 0.10 to 0.40 mm;
(F) wherein the capsule shell has a water content of 5.0 to 8.0%.

5. The soft capsule stable against mastication according to claim 2, wherein the capsule base is gelatin.

6. The soft capsule stable against mastication according to claim 2, wherein the plasticizer is glycerine which contains substantially no sorbitol or contains sorbitol in an amount of 20 parts by mass or less relative to 100 parts by mass of the capsule base.

7. The soft capsule stable against mastication according to claim 6, wherein the plasticizer is glycerine which contains substantially no sorbitol.

8. The soft capsule stable against mastication according to claim 2, wherein the capsule shell comprises gelatin as the capsule base and glycerine as the plasticizer which is contained in an amount of 25 to 40 parts by mass relative to 100 parts by mass of the gelatin, and has a water content of 5.0 to 9.0%.

9. The soft capsule stable against mastication according to claim 8, wherein the capsule shell comprises gelatin and glycerine which is contained in an amount of 30 parts by mass relative to 100 parts by mass of the gelatin, and has a water content of 5.0 to 8.0%.

10. A soft capsule stable against mastication which is easily disintegrated in the stomach, comprises (2R)-2-propyloctanoic acid, and is **characterized by**:

having a capsule shell comprising gelatin and glycerin which is contained in an amount of 30 parts by mass relative to 100 parts by mass of the gelatin, and having a water content of 5.0 to 8.0%, a shell thickness of 0.10 to 0.45 mm, a first seam thickness of 0.15 to 0.50 mm and a second seam thickness of 0.10 to 0.40 mm;
having a strength of 180 to 350 N by a cracking test; and
having a disintegration time of 5 to 8 minutes by the disintegration test stipulated in Japanese Pharmacopoeia.

11. The soft capsule stable against mastication according to claim 2 or 10, which comprises 50 to 400 mg of (2R)-2-propyloctanoic acid.

12. The soft capsule stable against mastication according to claim 11, which comprises 100 mg or 300 mg of (2R)-2-propyloctanoic acid.

13. The soft capsule stable against mastication according to claim 2 or 10, wherein dissolution delay accompanied by preservation is reduced.

14. The soft capsule stable against mastication according to claim 13, which has a dissolution rate of 90% or more 30 minutes after commencement of the dissolution test according to the second dissolution test stipulated in Japanese Pharmacopoeia, after preservation at room temperature for about one and half years.

15. The soft capsule stable against mastication according to claim 2 or 10, which is subjected to hermetically sealed packaging.

16. The soft capsule stable against mastication according to claim 15, wherein the hermetically sealed packaging is PTP packaging.

17. The soft capsule stable against mastication according to claim 2 or 10, which is an agent for prevention, treatment and/or suppression of symptom progression for neurodegenerative diseases, neuropathies or diseases in need of

nerve regeneration.

**18.** A method for prevention, treatment and/or suppression of symptom progression for neurodegenerative diseases, neuropathies or diseases in need of nerve regeneration, which comprises administering to a mammal an effective amount of the soft capsule stable against mastication according to claim 2 or 10.

**19.** Use of the soft capsule stable against mastication according to claim 2 or 10 for the manufacture of an agent for prevention, treatment and/or suppression of symptom progression for neurodegenerative diseases, neuropathies or diseases in need of nerve regeneration.

**20.** An agent for prevention, treatment and/or suppression of symptom progression for neurodegenerative diseases, neuropathies or diseases in need of nerve regeneration, which comprises the soft capsule stable against mastication according to claim 2 or 10.

**21.** A method for stabilizing a soft capsule against mastication, which comprises providing a soft capsule comprising (2R)-2-propyloctanoic acid or a salt thereof and having a capsule shell comprising (a) at least one capsule base selected from a protein, a saccharide, a biodegradable plastic and a hydrogenated fat and (b) a plasticizer, with at least one property selected from the following (A) to (E):

(A) wherein it has a strength of 150 to 400 N by a cracking test;
(B) wherein the capsule shell has a shell thickness of 0.05 to 0.50 mm;
(C) wherein the capsule shell has a first seam thickness of 0.10 to 0.55 mm;
(D) wherein the capsule shell has a second seam thickness of 0.05 to 0.50 mm;
(E) wherein the capsule shell has a water content of 5.0 to 9.0%.

**22.** The method according to claim 21, wherein the property is at least one selected from the following (A) to (E):

(A) wherein it has a strength of 180 to 350 N by a cracking test;
(B) wherein the capsule shell has a shell thickness of 0.10 to 0.45 mm;
(C) wherein the capsule shell has a first seam thickness of 0.15 to 0.50 mm;
(D) wherein the capsule shell has a second seam thickness of 0.10 to 0.40 mm;
(E) wherein the capsule shell has a water content of 5.0 to 8.0%.

**23.** A capsule stable against mastication which is easily disintegrated in the stomach, comprises an oral irritative compound, has a capsule shell comprising (a) at least one capsule base selected from a protein, a saccharide, a biodegradable plastic and a hydrogenated fat and (b) a plasticizer, and has at least one property selected from the following (A) to (F):

(A) wherein it has a strength of 150 to 400 N by a cracking test;
(B) wherein it has a disintegration time of 3 to 10 minutes by the disintegration test stipulated in Japanese Pharmacopoeia;
(C) wherein the capsule shell has a shell thickness of 0.05 to 0.50 mm;
(D) wherein the capsule shell has a first seam thickness of 0. 10 to 0.55 mm;
(E) wherein the capsule shell has a second seam thickness of 0.05 to 0.50 mm;
(F) wherein the capsule shell has a water content of 5.0 to 9.0%.

**24.** A method for stabilizing a soft capsule against mastication, which comprises providing a soft capsule comprising an oral irritative compound and having a capsule shell comprising (a) at least one capsule base selected from a protein, a saccharide, a biodegradable plastic and a hydrogenated fat and (b) a plasticizer, with at least one property selected from the following (A) to (E):

(A) wherein it has a strength of 150 to 400 N by a cracking test;
(B) wherein the capsule shell has a shell thickness of 0.05 to 0.50 mm;
(C) wherein the capsule shell has a first seam thickness of 0.10 to 0.55 mm;
(D) wherein the capsule shell has a second seam thickness of 0.05 to 0.50 mm;
(E) wherein the capsule shell has a water content of 5.0 to 9.0%.

## INTERNATIONAL SEARCH REPORT

International application No.
PCT/JP2005/011092

A. CLASSIFICATION OF SUBJECT MATTER
Int.Cl$^7$ A61K31/20, 9/48, 47/10, 47/42, A61P25/00

According to International Patent Classification (IPC) or to both national classification and IPC

B. FIELDS SEARCHED

Minimum documentation searched (classification system followed by classification symbols)
Int.Cl$^7$ A61K31/20, 9/48, 47/10, 47/42, A61P25/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)
CAOLD(STN), CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | JP 2002-97158 A (Ono Pharmaceutical Co., Ltd.), 02 April, 2002 (02.04.02), Particularly, Par. Nos. [0014], [0022] & EP 1174131 A1 & US 2002-32185 A | 1-17,19-24 |
| X<br>A | JP 5-65222 A (Fuji Capsule Kabushiki Kaisha), 19 March, 1993 (19.03.93), Particularly, tables 2, 3 (Family: none) | 23-24<br>1-17,19-22 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 06 July, 2005 (06.07.05) | 26 July, 2005 (26.07.05) |

| Name and mailing address of the ISA/ | Authorized officer |
|---|---|
| Japanese Patent Office | |
| Facsimile No. | Telephone No. |

Form PCT/ISA/210 (second sheet) (January 2004)

INTERNATIONAL SEARCH REPORT

International application No.
PCT/JP2005/011092

**Box No. II Observations where certain claims were found unsearchable (Continuation of item 2 of first sheet)**

This international search report has not been established in respect of certain claims under Article 17(2)(a) for the following reasons:

1. [X] Claims Nos.: 18
because they relate to subject matter not required to be searched by this Authority, namely:
The invention as set forth in claim 18 pertains to methods for treatment of the human body by therapy.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
While the invention of claims 1-17 and 19-22 is directed to soft capsules comprising (2R)-2-propyloctanoic acid as an active ingredient and having properties (A) to (F) set forth in claim 3, the invention of claims 23-24 is directed to soft capsules comprising an intraorally pungent compound as an active ingredient and having properties (A) to (F) set forth in claim 23. Consequently, it appears that the matter common to the two inventions is soft capsules with the properties (A) to (F). However, since the soft capsules with the properties (A) to (F), such as disintegration time of 3 to 10 min as measured by a disintegration testing method stipulated in Japanese
(continued to extra sheet)

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**
[ ] The additional search fees were accompanied by the applicant's protest.
[ ] No protest accompanied the payment of additional search fees.

Form PCT/ISA/210 (continuation of first sheet (2)) (January 2004)

INTERNATIONAL SEARCH REPORT

International application No.
PCT/JP2005/011092

Continuation of Box No.III of continuation of first sheet(2)

Pharmacopoeia, are publicly known as described in JP 5-65222 A, etc., this matter cannot be recognized as being a technical feature contributory over the prior art.

Form PCT/ISA/210 (extra sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0632008 A
- EP 1174131 A
- WO 9958513 A
- WO 0048982 A
- JP 3032447 B
- JP 3084354 B
- WO 03051852 A
- WO 04110972 A
- WO 0100663 A
- JP 11514333 T
- JP 2001500852 T


### Non-patent literature cited in the description


- **RICHARD C. LAROCK.** Comprehensive Organic Transformants: A Guide to Functional Group Preparations. John Wiley & Sons Inc, 1999
- Iyakuhin Tenkabuthu Jiten. Yakuji Nippo, 2000
- *Biochem. J.,* 1999, vol. 340 (1), 283-289